(19) Europäisches Patentamt
European Patent Office
Office européen des brevets

(11) Publication number: **0 071 483**
**B1**

(12) **EUROPEAN PATENT SPECIFICATION**

(45) Date of publication of patent specification: **02.01.85**

(51) Int. Cl.[4]: **C 07 C 97/18,** C 07 D 317/28 // A61K31/135, A61K31/335

(21) Application number: **82304021.7**

(22) Date of filing: **29.07.82**

(54) **Aminonaphthacene derivatives and their production.**

(30) Priority: **29.07.81 JP 119949/81**
**27.01.82 JP 12134/82**
**11.05.82 JP 79525/82**

(43) Date of publication of application:
**09.02.83 Bulletin 83/06**

(45) Publication of the grant of the patent:
**02.01.85 Bulletin 85/01**

(84) Designated Contracting States:
**AT BE CH DE FR GB IT LI NL SE**

(56) References cited:
**BE-A- 887 081**

**JOURNAL OF MEDICINAL CHEMISTRY, vol. 22, no. 8, August 1979, pages 922-926, E.M. ACTON et al.: "7-(Aminoethyl) ether and thioether of daunomycinone"**

(73) Proprietor: **SUMITOMO CHEMICAL COMPANY, LIMITED**
**15 Kitahama 5-chome Higashi-ku**
**Osaka-shi Osaka 541 (JP)**

(72) Inventor: **Ishizumi, Kikuo**
**10-1-126, Sonehigashi-machi, 2-chome**
**Toyonaka Osaka (JP)**
Inventor: **Muramatsu, Michihisa**
**4-1-1001 Miyahara 4-chome**
**Yodogawa-ku Osaka (JP)**
Inventor: **Sato, Hiromi**
**6-16 Hommachi 9-chome**
**Toyonaka Osaka (JP)**
Inventor: **Yoshida, Noboru**
**5-3-618 Higashiawaji 1-chome**
**Higashiyodogawa-ku Osaka (JP)**
Inventor: **Tanno, Norihiko**
**5-1-222 Hommachi, 9-chome**
**Toyonaka, Osaka (JP)**

(74) Representative: **Allard, Susan Joyce et al**
**BOULT, WADE & TENNANT 27 Furnival street**
**London EC4A 1PQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 071 483 B1

## Description

The present invention relates to aminonaphthacene derivatives and their production.

The aminonaphthacene derivatives are representable by the formula:

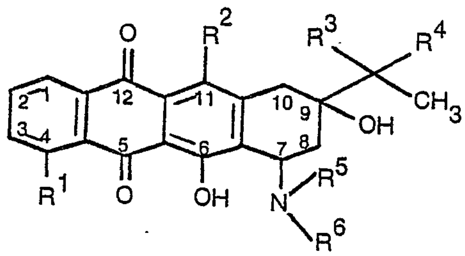

(I)

wherein $R^1$ is a hydrogen atom, a hydroxyl group or a lower alkoxy group, $R^2$ is a hydrogen atom or a hydroxyl group, $R^3$ and $R^4$ are each a lower alkoxy group or, when taken together, represent an ethylenedioxy group or an oxo group and $R^5$ and $R^6$ are each a hydrogen atom, a lower alkyl group or a group of the formula: —A—Q (in which A is an alkylene group optionally bearing at least one lower alkyl and Q is a hydrogen atom, a hydroxyl group, a lower alkoxy group or a group of the formula:

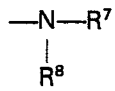

wherein $R^7$ and $R^8$ are each a hydrogen atom, a lower alkyl group, a benzyl group, a hydroxy(lower)alkyl group or an amino(lower)alkyl group or, when taken together with the adjacent nitrogen atom to which they are attached, represent a 5 to 7-membered nitrogen-containing ring optionally having an additional nitrogen or oxygen atom in the ring and optionally bearing any substituent on the ring) or, $R^5$ and $R^6$ when taken together with the adjacent nitrogen atom to which they are attached, represent a 5 to 7-membered nitrogen-containing ring optionally having an additional nitrogen or oxygen atom in the ring and optionally bearing any substituent on the ring.

By the term "lower alkyl" as used herein is meant a straight or branched alkyl group having not more than 4 carbon atoms and includes methyl, ethyl, n-propyl, isopropyl, n-butyl, isobutyl and t-butyl. By the term "lower alkoxy" as used herein is meant a straight or branched alkoxy group having not more than 4 carbon atoms and includes methoxy, ethoxy, n-propoxy, isopropoxy, n-butoxy, isobutoxy and t-butoxy. By the term "alkylene" as used herein is meant a straight or linear alkylene group having 2 to 8 carbon atoms which may be represented by the formula: $-(CH_2)_m-$ wherein m is an integer of 2 to 8. Such an alkylene group may be substituted with one or more lower alkyl groups on any methylene unit. By terms "hydroxy-(lower)alkyl" and "amino(lower)alkyl" as used herein are meant lower alkyl groups bearing a hydroxyl or amino group, respectively, on a carbon atom in the alkyl moiety. The 5 to 7-membered nitrogen-containing ring may be, for example, pyrrolidino, piperidino or hexamethyleneimino. The 5 to 7-membered nitrogen-containing ring having an additional nitrogen or oxygen atom in the ring may be, for example, morpholino or piperazino. Examples of the halogen atom are fluorine, chlorine, bromine and iodine.

The aminonaphthacene derivatives (I) have two asymmetric atoms, i.e. at the 7- and 9-positions, and are therefore intended to include the following four isomers:

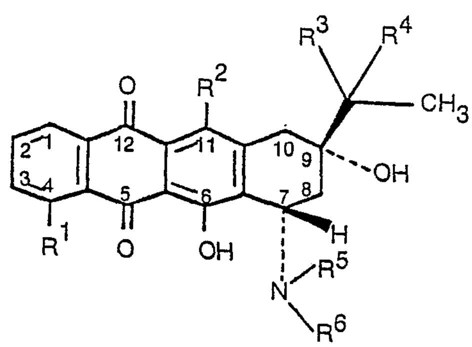

(7S, 9S)

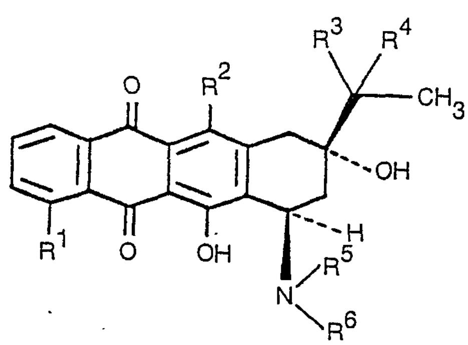

(7R, 9S)

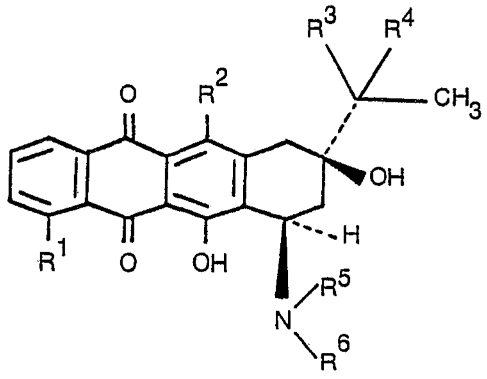

(7R, 9R)

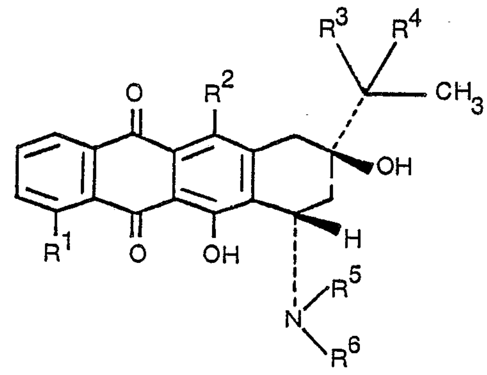

(7S, 9R)

among which the (7S, 9S) isomer is the most preferred.

Hitherto, some anthracyclines which are useful as anti-tumor agents have been known. In particular, adriamycin (hereinafter referred to as "ADR") and daunomycin (hereinafter referred to as "DMC") show strong anti-tumor activities and are used clinically. However, they produce marked side effects including cardiac toxicity. In addition, their separation and purification from natural sources is difficult and troublesome.

Recently, the chemical modification of ADR and DMC as well as the total synthesis of analogous compounds has been attempted. In particular, many studies have been made on the replacement of the sugar portion of ADR and DMC by another organic group, and a great number of compounds have been produced. However, none of these has been reported to exert a significant anti-tumor activity. For example, the 7-O-$\beta$-alanine ester and 7-O-$\beta$-aminoethyl ether of daunomycinone show only an uncertain effect against P388 leukemia in an animal test using mice (J. Med. Chem., *22*, 922-926).

It has now been unexpectedly found that the aminonaphthacene derivatives (I) show a remarkable anti-tumor activity. For example, they produce a significant growth inhibition against P388 tumor cells in the in vitro test as shown in Table 1. Further, for example, they produce a notable prolongation of life in the test using mice transplanted with P388 tumor cells.

TABLE 1

| Test Compound | Concen-tration ($\gamma$/ml) | Inhibition (%) |
|---|---|---|
| 7-(2-Hydroxyethyl)amino-9-acetyl-6,9,11-trihydroxy-5,7,8,9,10,12-hexahydronaphthacene-5,12-dione | 1 | 76.4 |
| 7-(2-Dimethylaminoethyl)amino-9-(1-ethylenedioxy)ethyl-6,9,11-trihydroxy-5,7,8,9,10,12-hexahydronaphthacene-5,12-dione | 1 | 72.1 |
| 7-(2-Dimethylaminoethyl)amino-9-acetyl-6,9,11-trihydroxy-5,7,8,9,10,12-hexahydronaphthacene-5,12-dione | 1 | 77.7 |
| 7(S)-(2-Dimethylaminoethyl)amino-9(S)-acetyl-6,9,11-trihydroxy-5,7,8,9,10,12-hexahydronaphthacene-5,12-dione | 1 | 71.2 |
| 7(S)-(2-Dimethylaminoethyl)amino-7-deoxydaunomycinone | 1 | 69.0 |
| Daunomycin | 1 | 68.3 |

Thus, the aminonaphthacene derivatives (I) are characteristic in having the structure comprising an anthracycline aglycon and an amino group introduced into the 7-position of the aglycon and yet showing a significant anti-tumor activity.

The aminonaphthacene derivatives (I) are useful as anti-tumor agents. They can be administered parenterally, orally or locally to warm-blooded animals and human beings in the form of conventional pharmaceutical preparations. For instance, they can be administered in the form of conventional solid pharmaceutical preparations such as tablets, capsules, powders or granules, or in the form of conventional liquid pharmaceutical preparations such as suspensions, emulsions or solutions. The daily dosage may vary depending upon the administration route and is usually between 0.1 to 100 mg/kg.

The aminonaphthacene derivatives (I), i.e. the 7-amino compounds, can be produced from the corresponding 7-H compound of the formula:

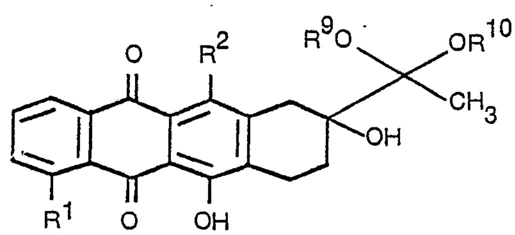

(II)

wherein $R^9$ and $R^{10}$ are each a lower alkyl group or, when taken together, represent an ethylene group and $R^1$ and $R^2$ are each as defined above by reacting the same with a halogenating agent to give the 7-X compound of the formula:

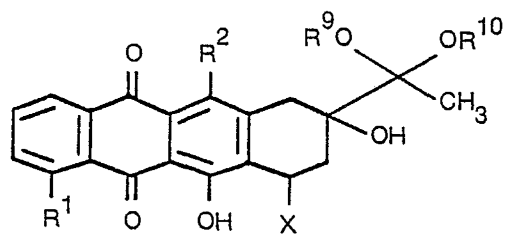

(III)

wherein X is a halogen atom and $R^1$, $R^2$, $R^9$ and $R^{10}$ are each as defined above and reacting the latter with an amine of the formula:

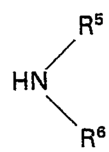

(C)

wherein $R^5$ and $R^6$ are each as defined above to give the 7-amino compound of the formula:

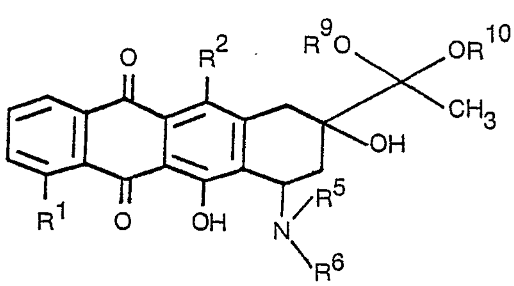

(Ia)

wherein $R^1$, $R^2$, $R^5$, $R^6$, $R^9$ and $R^{10}$ are each as defined above, optionally followed by elimination of the acetal group at the 13-position to give the 7-amino compound of the formula:

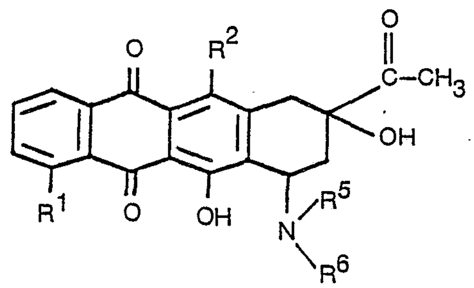

(Ib)

wherein $R^1$, $R^2$, $R^5$ and $R^6$ are each as defined above.

The starting 7-H compound is per se known and can be produced by subjecting the corresponding 9-acetyl compound to acetalation by a convention procedure.

The reaction of the 7-H compound (II) with a halogenating agent proceeds usually at a temperature of not lower than room temperature and can be accelerated by heating. When desired, a radical initiator (e.g. azobisisobutyronitrile, benzoyl peroxide) may be incorporated into the reaction system or a visible ray may be irradiated to the reaction system, whereby the reaction is promoted. Examples of the halogenating agent are bromine, chlorine, N-bromosuccinimide, N-chlorosuccinimide, etc. The reaction is normally effected in an inert solvent such as a halogenated hydrocarbon (e.g. carbon tetrachloride, chloroform, dichloromethane), an aromatic hydrocarbon (e.g. benzene, toluene), an aliphatic hydrogen (e.g. n-hexane, cyclohexane), an ether (e.g. diethyl ether, tetrahydrofuran, dioxane, diglyme), an amide (e.g. dimethylformamide), acetic acid or water, etc.

The reaction of the 7-X compound (III) with the amine (C) proceeds normally at room temperature but may be accelerated by heating. The reaction is usually carried out in an inert solvent such as an aromatic hydrocarbon (e.g. benzene, toluene), an ether (e.g. dimethyl ether, tetrahydrofuran), a halogenated hydrocarbon (e.g. carbon tetrachloride, chloroform, dichloromethane), an aliphatic hydrocarbon (e.g. n-hexane, cyclohexane) or an amide (e.g. dimethylformamide). When the amine (C) is a liquid at room temperature, it may be served as such as the reaction medium. In such case, the use of any other solvent is not necessarily needed.

Elimination of the acetal group at the 13-position as the optional step may be carried out by a per se conventional procedure such as hydrolysis under an acidic or basic condition or exchange in acetone under an acidic condition.

The thus produced amino compound (Ia) or (Ib) can be converted into its acid addition salt by treatment with an organic or inorganic acid. The control of the amount of the organic or inorganic acid to be used gives the salts in various molar proportions of the amino compound (Ia) or (Ib) and the organic or inorganic acid. Examples of the organic or inorganic acid are hydrochloric acid, hydrobromic acid, malic acid, citric acid, tartaric acid, etc.

Practical and presently preferred embodiments of the invention are illustratively shown in the following examples.

Example 1

(1). 9-(1,1-Ethylenedioxy)ethyl-6,9,11-trihydroxy-5,7,8,9,10,12-hexahydronaphthacene-5,12-dione (10.0 g) was dissolved in a mixture of chloroform (395 ml), carbon tetrachloride (980 ml) and water (890 ml) while heating, azobisisobutyronitrile (2.0 g) and bromine (8.0 g) were added thereto in order, and the resultant mixture was stirred under reflux for 1.5 hours. The reaction mixture was stirred in an ice bath for 1 hour, and the precipitated red orange crystals were collected by filtration, washed with water and dried under reduced pressure to give the crude product of 7-bromo-9-(1,1-ethylenedioxy)ethyl-6,9,11-trihydroxy-5,7,8,9,10,12-hexahydronaphthacene-5,12-dione. M.P., 196—235°C; IR (Nujol) $\nu cm^{-1}$: 3550, 1625, 1590. This product was used as such in the following step without purification.

(2) 7 - Bromo - 9 - (1,1 - ethylenedioxy)ethyl - 6,9,11 - trihydroxy - 5,7,8,9,10,12 - hexahydronaphthacene-5,12-dione (1.0 g) prepared in the foregoing step and N,N-dimethylethylenediamine (10 ml) were subjected to reaction at room temperature in nitrogen stream for 48 hours. N,N-Dimethylethylenediamine was removed by distillation under reduced pressure. The residue was dissolved in water (5 ml), adjusted to pH 8 with aqueous hydrochloric acid and extracted with chloroform. The chloroform extract was washed with water, dried over anhydrous sodium sulfate and concentrated. The resultant crude product was purified by silica gel chromatography using a mixture of chloroform and methanol (9:1) as an eluting solvent to give 7-(2-dimethylaminoethyl)amino-9 - (1,1 - ethylenedioxy)ethyl - 6,9,11 - trihydroxy - 5,7,8,9,10,12 - hexahydronaphthacene - 5,12 - dione as orange crystals. M.P., 239—244°C.

IR (Nujol) $\nu cm^{-1}$: 1630, 1595. NMR ($CDCl_3$) $\delta$: 1.5 (3H, s), 2.25 (6H, s), 2.0—3.15 (8H, m), 4.05 (4H, s), 4.17—4.33 (1H, bs), 7.65—7.90 (2H, m), 8.06—8.37 (2H, m). Mass spectrum (by the field desorption mass spectrometry): 483 $(M+1)^+$.

## Example 2

In the same manner as in Example 1 (2), 7-bromo-9-(1,1-ethylenedioxy)ethyl-6,9,11-trihydroxy-5,7,8,9,10,12-hexahydronaphthacene-5,12-dione (1.0 g) prepared in Example 1 (1) and N,N-dimethylethylenediamine (10 ml) were subjected to reaction. After removal of excessive N,N-dimethylethylenediamine by distillation under reduced pressure, the residue was crystallized from dichloromethane and ether to give 7-(2-dimethylaminoethyl)amino-9-(1,1-ethylenedioxy)-ethyl-6,9,11-trihydroxy-5,7,8,9,10,12-hexahydronaphthacene-5,12-dione as orange crystals. M.P., 242—245°C.

## Example 3

7-(2-Dimethylaminoethyl)amino-9-(1,1-ethylenedioxy)ethyl-6,9,11-trihydroxy - 5,7,8,9,10,12 - hexahydronaphthacene-5,12-dione (900 mg) prepared in Example 1 (2) was added to a mixture of acetone (90 ml) and conc. hydrochloric acid (36 ml), and the resultant mixture was heated at 50 to 60°C for 6 hours. After removal of acetone by distillation under reduced pressure, the residue was adjusted to pH 8.0 with aqueous sodium hydroxide solution and extracted with chloroform. The chloroform extract was purified by silica gel chromatography using a mixture of chloroform and methanol (9 : 1) as an eluting solvent to give 7-(2-dimethylaminoethyl)amino-9-acetyl-6,9,11-trihydroxy-5,7,8,9,10,12-hexahydronaphthacene-5,12-dione as orange crystals. M.P., 147°C (decomp.).

IR (Nujol) $\nu cm^{-1}$: 3330, 1720, 1630, 1590. NMR ($CDCl_3$): 1.10—1.90 (2H, m), 2.00—3.55 (8H, m), 2.23 (6H, s), 2.40 (3H, s), 4.30 (1H, bs), 7.65—7.95 (2H, m), 8.10—8.40 (2H, m). Mass spectrum: 440 $(M+1)^+$.

## Example 4

In the same manner as in Example 1 (2), 7-bromo-9-(1,1-ethylenedioxy)ethyl-6,9,11-trihydroxy-5,7,8,9,10,12-hexahydronaphthacene-5,12-dione (1.0 g) prepared in Example 1 (1) and ethanolamine (20 ml) were subjected to reaction to give 7-(2-hydroxyethyl)amino-9-(1,1-ethylenedioxy)ethyl-6,9,11-trihydroxy-5,7,8,9,10,12-hexahydronaphthacene-5,12-dione as orange crystals. M.P., 190°C (decomp.).

IR (Nujol) $\nu cm^{-1}$: 3530, 3330, 1630, 1590. NMR ($CDCl_3$) $\delta$: 1.43 (3H, s), 2.24—4.15 (8H, m), 4.03 (4H, s), 4.2—4.35 (1H, bs), 7.55—7.85 (2H, m), 7.9—8.2 (2H, m). Mass spectrum: 456 $(M+1)^+$.

## Example 5

In the same manner as in Example 3, 7-(2-hydroxyethyl)amino-9-(1,1-ethylenedioxy)ethyl-6,9,11-trihydroxy-5,7,8,9,10,12-hexahydronaphthacene-5,12-dione (1.0 g) prepared in Example 4 was subjected to reaction and post-treatment to give 7-(2-hydroxyethyl)amino-9-acetyl-6,9,11-trihydroxy-5,7,8,9,10,12-hexahydronaphthacene-5,12-dione. M.P., 125—130°C.

IR (Nujol) $\nu cm^{-1}$: 3400, 3300, 1705, 1620, 1590. NMR ($CDCl_3$) $\delta$: 1.65—2.25 (2H, m), 2.45 (3H, s), 2.90—3.15 (4H, m), 3.60—4.12 (2H, m), 4.30—4.45 (1H, bs), 7.75—7.82 (2H, m), 8.16—8.36 (2H, m).

## Example 6

In the same manner as in Example 2, 7-bromo-9-(1,1-ethylenedioxy)ethyl-6,9,11-trihydroxy-5,7,8,9,10,12-hexahydronaphthacene-5,12-dione (1.0 g) prepared in Example 1 (1) and N,N-dimethylpropylenediamine (20 ml) were subjected to reaction and post-treatment. The product was purified by silica gel chromatography using a mixture of chloroform and methanol (9 : 1) as an eluting solvent to give 7-(3-dimethylaminopropyl)amino-9-(1,1-ethylenedioxy)ethyl-6,9,11-trihydroxy-5,7,8,9,10,12-hexahydroxynaphthacene-5,12-dione as orange crystals. M.P., 239—244°C.

IR (Nujol) $\nu cm^{-1}$: 3300, 1620, 1580. NMR ($CDCl_3$) $\delta$: 1.47 (3H, s), 1.52—1.87 (4H, m), 2.20 (6H, s), 2.00—2.52 (2H, m), 2.70—3.22 (4H, m), 4.09 (4H, bs), 4.20—4.30 (1H, m), 7.70—7.93 (2H, m), 8.12—8.42 (2H, m).

## Example 7

7-(3-Dimethylaminopropyl)amino-9-(1,1-ethylenedioxy)ethyl-6,9,11-trihydroxy - 5,7,8,9,10,12 - hexahydronaphthacene-5,12-dione (115 mg) prepared in Example 6 was dissolved in acetone (115 ml), conc. hydrochloric acid (40 ml) was added thereto, and the resultant mixture was subjected to reaction at room temperature for 6.5 hours. After removal of the solvent by distillation under reduced pressure, the residue was admixed with water, adjusted to pH 7.9—8.0 and extracted with chloroform. The extract was purified by silica gel chromatography using a mixture of chloroform and methanol (9 : 1) as an eluting solvent to give 7-(3-dimethylaminopropyl)amino-9-acetyl-6,9,11-trihydroxy-5,7,8,9,10,12-hexahydronaphthacene-5,12-dione as a dark brown semi-solid.

IR (Nujol) $\nu cm^{-1}$: 3200—3600, 1710, 1695, 1630, 1595. NMR ($CDCl_3$) $\delta$: 1.40—2.06 (4H, m), 2.22 (6H, s), 2.06—2.63 (5H, m), 2.63—3.21 (4H, m), 4.10—4.34 (1H, m), 7.50—7.90 (2H, m), 7.98—8.37 (3H, m).

Example 8

In the same manner as in Example 2, 7-bromo-9-(1,1-ethylenedioxy)ethyl-6,9,11-trihydroxy-5,7,8,9,10,12-hexahydronaphthacene-5,12-dione (1.0 g) prepared in Example 1 (1) and N,N-diethylethylenediamine (20 ml) were subjected to reaction. The product was purified by silica gel chromatography using a mixture of chloroform and methanol (9 : 1) as an eluting solvent to give 7-(2-diethylaminoethyl) - amino - 9 - (1,1 - ethylenedioxy)ethyl - 6,9,11 - trihydroxy - 5,7,8,9,10,12 - hexahydronaphthacene-5,12-dione as dark orange crystals. M.P., 120—126°C.

IR (Nujol) $\nu cm^{-1}$: 3300, 1620, 1585. NMR ($CDCl_3$) $\delta$: 1.01 (3H, t), 1.50 (3H, s), 2.18—3.34 (12H, m), 4.08 (4H, bs), 4.19—4.33 (1H, m), 7.63—7.9 (2H, m), 8.18—8.42 (2H, m).

Example 9

7-(2-Diethylaminoethyl)amino-9-(1,1 - ethylenedioxy)ethyl - 6,9,11 - trihydroxy - 5,7,8,9,10,12 - hexahydronaphthacene-5,12-dione (150 mg) prepared in Example 8 was dissolved in acetone (150 ml), conc. hydrochloric acid (60 ml) was added thereto, and the resultant mixture was stirred at room temperature for 5 hours. After removal of acetone by distillation, water was added to the residue, the pH was adjusted to 8.0 and the product was extracted with chloroform. The extract was purified by silica gel chromatography using a mixture of chloroform and methanol (9 : 1) as an eluting solvent to give 7-(2-diethylaminoethyl)amino-9-acetyl-6,9,11-trihydroxy-5,7,8,9,10,12-hexahydronaphthacene-5,12-dione as a blackish brown semi-solid.

IR (Nujol) $\nu cm^{-1}$: 3300, 1710, 1625, 1590. NMR ($CDCl_3$) $\delta$: 0.99 (6H, t), 2.40 (3H, s), 2.10—3.33 (8H, m), 4.11—4.36 (1H, m), 7.60—7.88 (2H, m). Mass spectrum: 467 $(M+1)^+$.

Example 10

7-Bromo-9-(1,1-ethylenedioxy)ethyl-6,9,11-trihydroxy-5,7,8,9,10,12-hexahydronaphthacene-5,-12-dione (1.0 g) prepared in Example 1 (1) and ethylenediamine (16 ml) were subjected to reaction at room temperature under nitrogen stream for 48 hours. Excess of the ethylenediamine was distilled off under reduced pressure, cold water (100 ml) was added thereto, and the pH was adjusted to 7.2. The product was extracted with chloroform and purified by silica gel chromatography using a mixture of chloroform and methanol (9 : 1) as an eluting solvent to give 7-(2-aminoethyl)amino-9-(1,1-ethylenedioxy)ethyl-6,9,11-trihydroxy-5,7,8,9,10,12-hexahydronaphthacene-5,12-dione as an orange semi-solid.

IR (Nujol) $\nu cm^{-1}$: 3300—3400, 1620, 1590. Mass spectrum: 455 $(M+1)^+$.

Example 11

7-Bromo-9-(1,1-ethylenedioxy)ethyl-6,9,11-trihydroxy-5,7,8,9,10,12-hexahydronaphthacene-5,-12-dione (1.0 g) prepared in Example 1 (1) and N-(2-aminoethyl)ethanolamine (20 ml) were subjected to reaction at room temperature in nitrogen stream for 48 hours. Excess of the amine was eliminated by distillation under reduced pressure, water was added to the residue, and the pH was adjusted to 8.0. The product was extracted with chloroform and purified by silica gel chromatography using a mixture of chloroform and methanol (9 : 1) as an eluting solvent to give 7-(2-(2-hydroxyethyl)aminoethyl)amino-9-(1,1 - ethylenedioxy)ethyl-6,9,11-trihydroxy - 5,7,8,9,10,12 - hexahydronaphthacene-5,12-dione as a dark brown semi-solid.

IR (Nujol) $\nu cm^{-1}$: 3600—3100, 1620, 1590. NMR ($CDCl_3$) $\delta$: 1.50 (3H, s), 2.10—3.83 (12H, m), 4.07 (4H, s), 4.16—4.36 (1H, m), 7.56—7.86 (2H, m), 8.00—8.30 (2H, m). Mass spectrum: 499 $(M+1)^+$.

Example 12

(1) 9(R)-(1,1-Ethylenedioxy)ethyl-6,9,11-trihydroxy-5,7,8,9,10,12-hexahydronaphthacene-5,12-dione (1.35 g) produced from 9(R)-acetyl-6,9,11-trihydroxy-5,7,8,9,10,12-hexahydronaphthacene-5,12-dione ($[\alpha]_D^{20}$ −87° (C = 0.1 in chloroform)) was dissolved in a mixture of chloroform (59 ml), carbon tetrachloride (130 ml) and water (118 ml) while heating. Azobisisobutyronitrile (300 mg) and bromine (1.24 g) were added thereto in order. The resultant mixture was heated under reflux for 1 hour. From the reaction mixture, an organic phase was separated, washed with aqueous sodium hyposulfite solution and water, dried over anhydrous sodium sulfate and concentrated to give the crude product of 7-bromo-9(S)-(1,1-ethylenedioxy)ethyl-6,9,11-trihydroxy-5,7,8,9,10,12-hexahydronaphthacene-5,12-dione as red powder. This product was used in the subsequent step without purification.

(2) 7 - Bromo - 9(S) - (1,1 - ethylenedioxy)ethyl - 6,9,11 - trihydroxy - 5,7,8,9,10,12 - hexahydronaphthacene-5,12-dione (500 mg) prepared in the foregoing step was reacted with N,N-dimethylethylenediamine (5 ml) in the same manner as in Example 1 (2). The resultant mixture was subjected to post-treatment to give the following products:

7(S) - (2 - Dimethylaminoethyl)amino - 9(S) - (1,1 - ethylenedioxy)ethyl - 6,9,11 - trihydroxy - 5,7,8,9,10,12 - hexahydronaphthacene - 5,12 - dione as orange crystals. M.P., 165—169°C. $[\alpha]_D^{20}$ +120° (C = 0.1 in chloroform).

NMR ($CDCl_3$) $\delta$: 1.5 (3H, s), 2.25 (6H, s), 2.00—3.15 (8H, m), 4.05 (4H, s), 4.25 (1H, brs, $\nu 1/2$ = 8.3Hz), 7.65—7.90 (2H, m), 8.06—8.37 (2H, m). Mass spectrum: 483 $(M+1)^+$.

7(R) - (2 - Dimethylaminoethyl)amino 9(S) - (1,1 - ethylenedioxy)ethyl - 6,9,11 - trihydroxy - 5,7,8,9,10,12-hexahydronaphthacene-5,12-dione as brown semi-solid. $[\alpha]_D^{20}$ −316° (C = 0.1 in chloroform).

NMR ($CDCl_3$) δ: 1.45 (3H, s), 1.60—3.37 (9H, m), 2.18 (6H, s), 4.07 (4H, s), 4.47 (brt, $J_1$=$J_2$=9Hz, ν*l*/2=20.1Hz), 7.78 (2H, m), 8.29 (2H, m). Mass spectrum: 483 $(M+1)^{+\cdot}$

Example 13

In the same manner as in Example 3, 7(S)-(2-dimethylaminoethyl)amino - 9(S) - (1,1 - ethylenedioxy)ethyl - 6,9,11 - trihydroxy - 5,7,8,9,10,12 - hexahydronaphthacene - 5,12 - dione prepared in Example 12 was subjected to reaction and post-treatment to give 9(S) - acetyl - 7(S) - (2 - dimethylaminoethyl)amino - 6,9,11 - trihydroxy - 5,7,8,9,10,12 - hexahydronaphthacene - 5,12 - dione as orange crystals. M.P., 116—120°C. $[\alpha]_D^{20}$ +160° (C = 0.11 in chloroform).

NMR ($CDCl_3$) δ: 1.15—1.88 (2H, m), 2.26 (6H, s), 2.41 (3H, s), 2.13—3.32 (8H, m), 4.27 (1H, brs), 7.65—7.92 (2H, m), 8.08—8.38 (2H, m).

Hydrochloride, M.P., 218—221°C.

Example 14

In the same manner as in Example 1 (2), 7-bromo-9-(1,1-ethylenedioxy)ethyl-6,9,11-trihydroxy-5,7,8,9,10,12-hexahydronaphthacene-5,12-dione (1.0 g) prepared in Example 1 (1) and N-methylpiperazine (10 ml) were subjected to reaction. The product was purified by silica gel chromatography using a mixture of chloroform and methanol (9 : 1) as an eluting solvent to give 7-N'-methylpiperazinyl - 9 - (1,1-ethylenedioxy)ethyl - 6,9,11 - trihydroxy - 5,7,8,9,10,12 - hexahydronaphthacene - 5,12 - dione as an orange semi-solid.

IR (Nujol) ν$cm^{-1}$: 3400, 1620, 1590. NMR ($CDCl_3$) δ: 1.48 (3H, s), 1.68 (1H, m), 2.07—3.23 (12H, m), 2.22 (3H, s), 4.02 (4H, s), 4.25 (1H, s), 7.63—7.90 (2H, m), 8.13—8.43 (2H, m), 13.32 (1H, s), 13.63 (1H, s).

Example 15

7-Bromo-9-(1,1-ethylenedioxy)ethyl-6,9,11-trihydroxy-5,7,8,9,10,12-hexahydronaphthacene-5,12-dione (1.0 g) prepared in Example 1 (1) and N-β-aminoethylmorpholine (20 ml) were subjected to reaction at a temperature of 0 to 5°C for 12 hours. The reaction mixture was poured into ice water, adjusted to pH 8.0 and extracted with dichloromethane. The extract was purified by silica gel chromatography using a mixture of chloroform and methanol (9 : 1) to give the following products:

A stereo-isomer of 7-(1-morpholinoethyl)amino-9-(1,1-ethylenedioxy)ethyl-6,9,11-trihydroxy-5,7,8,9,10,12-hexahydronaphthacene-5,12-dione as orange crystals. M.P., 109—116°C.

IR (Nujol) ν$cm^{-1}$: 3350, 1620, 1590. NMR ($CDCl_3$) δ: 1.43—1.73 (1H, m), 1.48 (3H, s), 2.10—3.40 (13H, m), 3.57—3.77 (4H, m), 4.05 (4H, s), 4.29 (1H, brs, ν*l*/2=8.3Hz), 7.73—7.93 (2H, m), 8.20—8.45 (2H, m).

Another stereo-isomer of the said compound as orange crystals. M.P., 122—130°C.

IR (Nujol) ν$cm^{-1}$: 3400, 1620, 1590. NMR ($CDCl_3$) δ: 1.45 (3H, s), 1.77—3.43 (13H, m), 3.57—3.83 (4H, m), 4.08 (4H, s), 4.50 (1H, m, ν*l*/2=21Hz), 7.67—7.93 (2H, m), 8.13—8.40 (2H, m).

Reference Example 1

A mixture of 7-deoxydaunomycinone (239 mg) (prepared from daunomycin hydrochloride according to the method as described in J. Org. Chem., *42*, 3657 (1977)), benzene (15 ml), ethylene glycol (0.5 ml) and p-toluene-sulfonic acid (10 mg) was heated under reflux for 3 hours, during which the by-produced water was eliminated from the reaction system. After cooling, the precipitated orange crystals of 7-deoxydaunomycinone-13-ethyleneacetal (M.P., 279—282°C) were collected by filtration.

Example 16

(1) A mixture of 7-deoxydaunomycinone-13-ethyleneacetal (340 mg) prepared in Reference Example 1, chloroform (15 ml), carbon tetrachloride (30 ml) and water (20 ml) was heated under reflux, and a solution of bromine (300 mg) in carbon tetrachloride (4 ml) was dropwise added thereto. A half portion of 72 mg of azobisisobutyronitrile and the reminder were added to the reaction system respectively at the initial stage and at the final stage. After the dropwise addition was completed, heating under reflux was continued for 30 minutes. The reaction mixture was cooled to room temperature, and the organic phase was separated, washed with dilute sodium hyposulfite solution and water in order, dried over anhydrous sodium sulfate and concentrated under reduced pressure to give 7-bromo-7-deoxydaunomycinone-13-ethyleneacetal as red crystals. This product was used as such in the subsequent step without purification.

(2) In the same manner as in Example 1 (2), 7-bromo-7-deoxydaunomycinone-13-ethyleneacetal (100 mg) prepared in the foregoing step and N,N-dimethylethylenediamine (5 ml) were subjected to reaction and post-treatment. The product was purified by thin layer chromatography using silica gel and a mixture of dichloromethane and methanol (9 : 1) as an eluting solvent to give 7-deoxy-7-(2-dimethylaminoethyl)aminodaunomycinone-13-ethyleneacetal as orange crystals. M.P., 185—188°C.

NMR ($CDCl_3$) δ: 1.35—1.78 (1H, m), 1.49 (3H, s), 2.07—3.42 (10H, m), 2.21 (3H, s), 4.07 (4H, brs), 4.33 (1H, brs), 7.40 (1H, brs), 7.65—7.88 (1H, m), 7.95—8.15 (1H, m). Mass spectrum: 513 $(M+1)^+$.

## Example 17

7-Deoxy-7(S)-(2-dimethylaminoethyl)aminodaunomycinone-13-ethyleneacetal (60 mg) prepared in Example 16 (2) was dissolved in a mixture of acetone (60 ml) and conc. hydrochloric acid (30 ml), and the resultant mixture was stirred at room temperature for 5 hours. After removal of the acetone by distillation under reduced pressure, the residue was adjusted to pH 8.0 and extracted with chloroform. The extract was dried and concentrated under reduced pressure to give 7-deoxy-7(S)-(2-dimethylaminoethyl)aminodaunomycinone as an orange solid.

NMR ($CDCl_3$) δ: 1.57—1.87 (1H, m), 2.11—3.33 (7H, m), 2.22 (6H, s), 2.38 (3H, s), 4.03 (3H, s), 4.29 (1H, brs), 7.31—8.02 (3H, m). Mass spectrum: 469 $(M+1)^+$.

## Reference Example 2

(1) A mixture of 7-deoxydaunomycine (1.06 g) (prepared from daunomycin hydrochloride by the method as described in J. Org. Chem., *42*, 3657 (1977)), dry dichloromethane (110.8 ml) and anhydrous aluminum chloride (3.32 g) was heated under reflux for 2 hours. The reaction mixture was dropwise added to a cold solution of oxalic acid (1.7 g) in water (60 ml). The resultant mixture was stirred at room temperature for 1 hour. The organic phase was separated, washed with water, dried over anhydrous sodium sulfate and concentrated under reduced pressure to give 7-deoxycarminomycinone as red brown crystals. M.P., 247—249°C.

IR (Nujol) $\nu cm^{-1}$: 3480, 1700, 1620, 1600. NMR ($CDCl_3$) δ: 1.7—2.0 (2H, m), 2.2 (3H, s), 2.7—3.0 (4H, m), 7.15—7.4 (1H, m), 7.5—7.95 (2H, m), 11.8—12.3 (1H, m), 12.3—12.7 (1H, m), 13.3—13.6 (1H, m). Mass spectrum: 368 $(M)^+$.

(2) A mixture of 7-deoxycarminomycinone (500 mg) prepared in the foregoing step, dry benzene (24 ml), ethylene glycol (0.72 ml) and p-toluenesulfonic acid (24 mg) was heated under reflux for 4 hours, during which the by-produced water was eliminated from the reaction system. After cooling, the precipitated orange crystals of 7-deoxycarminomycinone-13-ethyleneacetal were collected by filtration. M.P., 257—259°C.

IR (Nujol) $\nu cm^{-1}$: 3440, 1600, 1570. NMR ($CDCl_3$) δ: 1.45 (3H, s), 1.85—2.15 (2H, m), 2.65—3.15 (4H, m), 4.08 (4H, s), 7.2—8.0 (3H, m). Mass spectrum: 412 $(M)^+$.

## Example 18

(1) 7-Deoxycarminomycinone-13-ethyleneacetal (270 mg) prepared in Reference Example 2 (2) was dissolved in dry carbon tetrachloride (1080 ml), and a solution of bromine (230 mg) in carbon tetrachloride (10 ml) was dropwise added thereto. A half portion of 28.35 mg of azobisisobutyronitrile and the reminder were added to the reaction mixture respectively at the initial stage and at the complete stage. After the dropwise addition was completed, the reaction mixture was heated under reflux for 1 hour. The solvent was removed by distillation under reduced pressure to give 7-bromo-7-deoxycarminomycinone-13-ethyleneacetal as red brown crystals. This product was used as such in the subsequent step without purificatrion.

(2) 7-Bromo-7-deoxycarminomycinone-13-ethyleneacetal (310 mg) prepared in the foregoing step and N,N-dimethylethylenediamine (10 ml) were subjected to reaction at −40 to −50°C in nitrogen stream for 1 hour. Excess of the amine was eliminated by distillation under reduced pressure. The residue was poured into ice water, adjusted to pH 8.0 and extracted with dichloromethane. The extract was concentrated and purified by silica gel chromatography using a mixture of dichloromethane and methanol (9 : 1) as an eluting solvent to give 7-deoxy-7(S)-(2-dimethylaminoethyl)amino-carminomycinone-13-ethyleneacetal as orange crystals. M.P. 151—153°C.

IR (Nujol) $\nu cm^{-1}$: 3100—3350, 1605, 1580. NMR ($CDCl_3$) δ: 1.45 (3H, s), 1.50—1.66 (1H, m), 2.2 (6H, s), 2.0—3.2 (8H, m), 4.05 (4H, s), 4.2—4.35 (1H, m, νl/2=7.5Hz), 7.5—7.9 (3H, m). Mass spectrum: 500 $(M+2)^+$.

## Example 19

7-Deoxy-7(S)-(2-dimethylaminoethyl)aminocarminomycinone-13-ethyleneacetal (55 mg) prepared in Example 18 (2) was dissolved in a mixture of acetone (55 ml) and conc. hydrochloric acid (22 ml), and the resultant mixture was stirred at room temperature for 16 hours. After removal of the acetone by distillation under reduced pressure, the residue was washed with dichloromethane and adjusted to pH 8.0. The product was extracted with dichloromethane, and the extract was dried over anhydrous sodium sulfate and concentrated to give 7-deoxy-7(S)-(2-dimethylaminoethyl)amino-carminomycinone as orange crystals. M.P., 244—246°C.

IR (Nujol) $\nu cm^{-1}$: 3100—3500, 1718, 1600. NMR ($CDCl_3$) δ: 1.6—1.78 (1H; m), 2.21 (6H, s), 2.38 (3H, s), 2.0—2.1 (8H, m), 4.1—4.3 (1H, m, νl/2=9Hz), 7.1—7.8 (3H, m). Mass spectrum: 454 $(M+1)^+$.

Hydrochloride, M.P., 155—157°C.

Example 20

(1) A mixture of 9 - (1,1 - ethylenedioxy)ethyl - 6,9 - dihydroxy - 5,7,8,9,10,12 - hexahydronaphthacene - 5,12 - dione (657 mg), chloroform (33 ml), carbon tetrachloride (13 ml) and water (26 ml) was stirred at room temperature, and bromine (530 mg) and azobisisobutyronitrile (130 mg) were added thereto, and the resulting mixture was stirred at the same temperature as above for 1 hour. From the reaction mixture, the organic phase was separated, washed with water, dried over anhydrous sodium sulfate and concentrated. The residue was crystallized from ether to give 7 - bromo - 9 - (1,1 - ethylenedioxy)ethyl - 6,9 - dihydroxy - 5,7,8,9,10,12 - hexahydronaphthacene - 5,12 - dione (576 mg). M.P., 173—176°C.

IR (Nujol) $\nu cm^{-1}$: 3475, 1670, 1630, 1590.

(2) 7 - Bromo - 9 - (1,1 - ethylenedioxy)ethyl - 6,9 - dihydroxy - 5,7,8,9,10,12 - hexahydronaphthacene - 5,12 - dione (571 mg) prepared in the foregoing step and N,N-dimethylethylenediamine (10 ml) were subjected to reaction in nitrogen stream while cooling with ice for 1 hour. Excess of the N,N-dimethylethylenediamine was eliminated by distillation under reduced pressure. The residue was dissolved in dichloromethane (2 ml) and ether (4 ml) was added thereto, whereby 7 - (2 - dimethylaminoethyl)amino - 9 - (1,1 - ethylenedioxy)ethyl - 6,9 - dihydroxy - 5,7,8,9,10,12 - hexahydronaphthacene - 5,12 - dione was obtained as yellow crystals (124 mg). M.P., 192—196°C.

IR (Nujol) $\nu cm^{-1}$: 1670, 1630, 1590, 1575. NMR ($CDCl_3$) $\delta$: 1.44 (3H, s), 1.62—3.05 (6H, m), 2.24 (6H, s), 3.13 (2H, bs), 4.03 (4H, bs), 4.32 (1H, bs), 7.60 (1H, s), 7.75 (2H, m), 8.27 (2H, m).

Example 21

7 - (2 - Dimethylaminoethyl)amino - 9 - (1,1 - ethylenedioxy)ethyl - 6,9 - dihydroxy - 5,7,8,9,10,12 - hexahydronaphthacene - 5,12 - dione (60 mg) prepared in Example 20 (2) was added to a mixture of acetone (60 ml) and conc. hydrochloric acid (24 ml), and the resultant mixture was heated under reflux for 1 hour. After removal of acetone by distillation under reduced pressure, the water layer was washed with dichloromethane, adjusted to pH 8.0 with aqueous sodium hydroxide solution and extracted with dichloromethane. The extract was purified by silica gel chromatography using a mixture of chloroform and methanol (9:1) to give 7 - (2 - dimethylaminoethyl)amino - 9 - acetyl - 6,9 - dihydroxy - 5,7,8,9,10,12 - hexahydronaphthacene - 5,12 - dione (55 mg). M.P. 170—172°C.

IR (Nujol) $\nu cm^{-1}$: 3300, 1715, 1675, 1630, 1595, 1575. NMR ($CDCl_3$) $\delta$: 1.60—3.28 (9H, m), 3.23 (6H, s), 2.39 (3H, s), 4.32 (1H, bs), 7.47 (1H, s), 7.75 (2H, m), 8.23 (2H, s).

Hydrochloride, M.P., 207—216°C.

**Claims for the Contracting States: BE CH DE FR GB IT LI NL SE**

1. A compound of the formula:

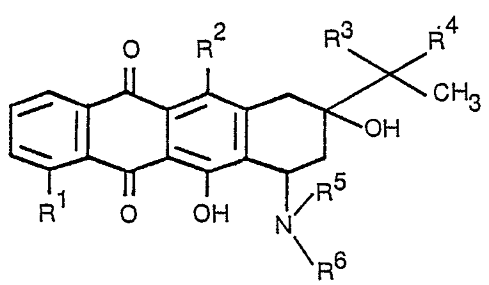

(I)

wherein $R^1$ is a hydrogen atom, a hydroxyl group or a lower alkoxy group, $R^2$ is a hydrogen atom or a hydroxyl group, $R^3$ and $R^4$ are each a lower alkoxy group or, when taken together, represent an ethylenedioxy group or an oxo group and $R^5$ and $R^6$ are each a hydrogen atom, a lower alkyl group or a group of the formula: —A—Q (in which A is an alkylene group optionally bearing at least one lower alkyl and Q is a hydrogen atom, a hydroxyl group, a lower alkoxy group or a group of the formula:

—N—$R^7$
|
$R^8$

wherein $R^7$ and $R^8$ are each a hydrogen atom, a lower alkyl group, a benzyl group, a hydroxy(lower)alkyl group or an amino(lower)alkyl group or, when taken together with the adjacent nitrogen atom to which they are attached, represent a 5 to 7-membered nitrogen-containing ring optionally having an

additional nitrogen or oxygen atom in the ring and optionally bearing any substituent on the ring) or, $R^5$ and $R^6$, when taken together with the adjacent nitrogen atom to which they are attached, represent a 5 to 7-membered nitrogen-containing ring optionally having an additional nitrogen or oxygen atom in the ring and optionally bearing any substituent on the ring, or its acid addition salt.

2. A compound as claimed in Claim 1 wherein $R^3$ and $R^4$ are each lower alkoxy or, when taken together, represent an ethylenedioxy group.

3. A compound as claimed in Claim 1 wherein $R^3$ and $R^4$ are taken together to represent an oxo group.

4. A compound as claimed in Claim 1 wherein $R^3$ and $R^4$ are taken together to represent an oxo group, $R^5$ is a hydrogen atom and $R^6$ is a group of the formula: —A—Q.

5. A compound as claimed in Claim 1, wherein $R^3$ and $R^4$ are taken together to represent an oxo group and $R^5$ is a hydrogen atom and $R^6$ is a group of the formula:

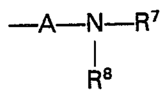

6. A compound as claimed in Claim 5 wherein $R^7$ and $R^8$ are each a hydrogen atom, a lower alkyl group, a benzyl group, a hydroxy (lower)alkyl group or an amino(lower)alkyl group.

7. A compound as claimed in Claim 5 wherein $R^7$ and $R^8$ are each a hydrogen atom or a lower alkyl group.

8. A compound as claimed in Claim 6 wherein $R^1$ is a hydrogen atom and $R^2$ is a hydroxyl group.

9. A compound as claimed in Claim 6 wherein $R^1$ is a hydroxyl group and $R^2$ is a hydroxyl group.

10. A compound as claimed in any one of Claim 1 to 9 wherein the configurations at the 7- and 9-positions are the S-configurations.

11. A process for preparing a compound of the formula:

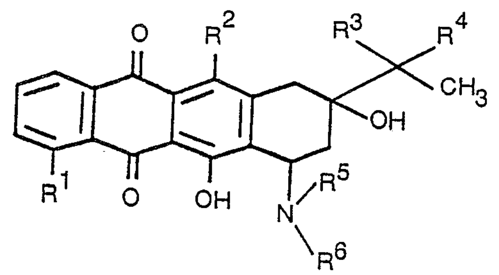

(I)

wherein $R^1$, $R^2$, $R^3$, $R^4$, $R^5$ and $R^6$ are as defined in Claim 1 or its acid addition salt, which process comprises reacting a compound of the formula:

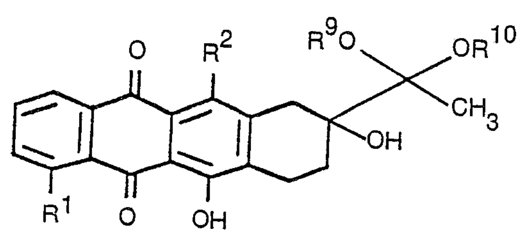

(II)

wherein $R^9$ and $R^{10}$ are each a lower alkyl group or, when taken together, represent an ethylene group and $R^1$ and $R^2$ are each as defined above with a halogenating agent to give the 7—X compound of the formula:

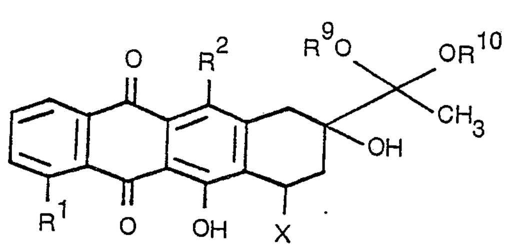

(III)

wherein X is a halogen atom and $R^1$, $R^2$, $R^9$ and $R^{10}$ are each as defined above and reacting the latter with an amine of the formula:

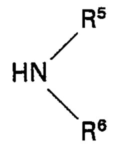

(C)

wherein $R^5$ and $R^6$ are each as defined above to give the 7-amino compound of the formula:

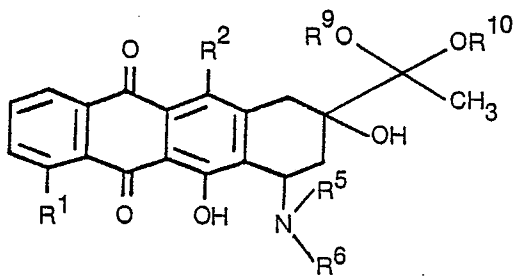

(Ia)

wherein $R^1$, $R^2$, $R^5$, $R^6$, $R^9$ and $R^{10}$ are each as defined above, optionally followed by elimination of the acetal group at the 13-position to give the 7-amino compound of the formula:

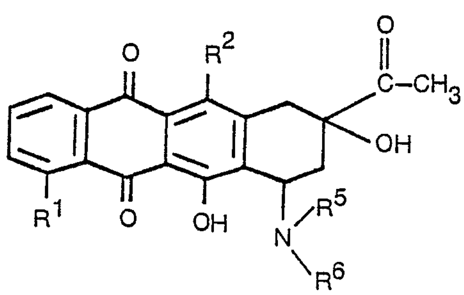

(Ib)

wherein $R^1$, $R^2$, $R^5$ and $R^6$ are each as defined above.

12. A process for preparing a compound of the formula:

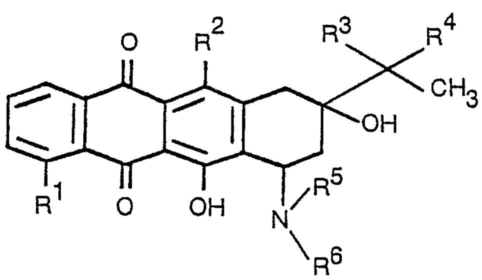

(I)

wherein $R^1$, $R^2$, $R^3$, $R^4$, $R^5$ and $R^6$ are as defined in Claim 1 or its acid additional salt, which process comprises reacting a compound of the formula:

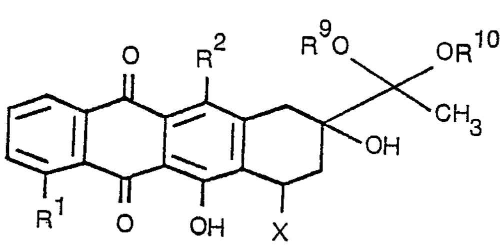

(III)

wherein X is a halogen atom, $R^9$ and $R^{10}$ are each a lower alkyl group or, when taken together, represent an ethylene group and $R^1$ and $R^2$ are each as defined above with an amine of the formula:

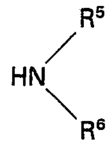

(C)

wherein $R^5$ and $R^6$ are each as defined above to give the 7-amino compound of the formula:

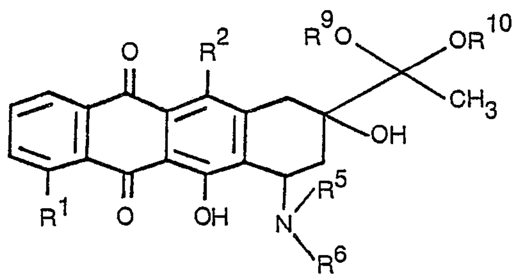

(Ia)

wherein $R^1$, $R^2$, $R^5$, $R^6$, $R^9$ and $R^{10}$ are each as defined above, optionally followed by elimination of the acetal group at the 13-position to give the 7-amino compound of the formula:

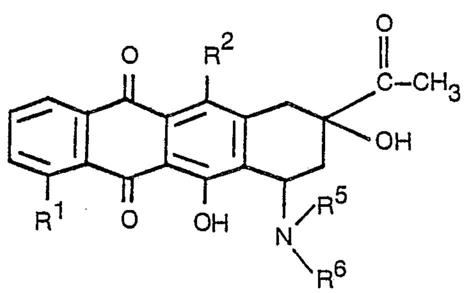

(Ib)

wherein $R^1$, $R^2$, $R^5$ and $R^6$ are each as defined above.

**Claims for the Contracting State: AT**

1. A process for preparing a compound of the formula:

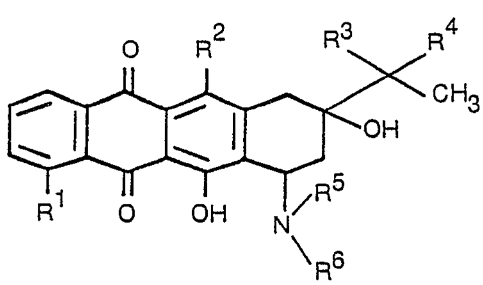

(I)

wherein $R^1$ is a hydrogen atom, a hydroxyl group or a lower alkoxy group, $R^2$ is a hydrogen atom or a hydroxyl group, $R^3$ and $R^4$ are each a lower alkoxy group or, when taken together, represent an ethylenedioxy group or an oxo group and $R^5$ and $R^6$ are each a hydrogen atom, a lower alkyl group or a group of the formula: —A—Q (in which A is an alkylene group optionally bearing at least one lower alkyl and Q is a hydrogen atom, a hydroxyl group, a lower alkoxy group or a group of the formula:

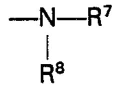

wherein $R^7$ and $R^8$ are each a hydrogen atom, a lower alkyl group, a benzyl group, a hydroxy(lower)alkyl group or an amino(lower)alkyl group or, when taken together with the adjacent nitrogen atom to which they are attached, represent a 5 to 7-membered nitrogen-containing ring optionally having an additional nitrogen or oxygen atom in the ring and optionally bearing any substituent on the ring) or, $R^5$ and $R^6$ when taken together with the adjacent nitrogen atom to which they are attached, represent a 5 to 7-membered nitrogen-containing ring optionally having an additional nitrogen or oxygen atom in the ring and optionally bearing any substituent on the ring, or its acid addition salt, which process comprises reacting a compound of the formula:

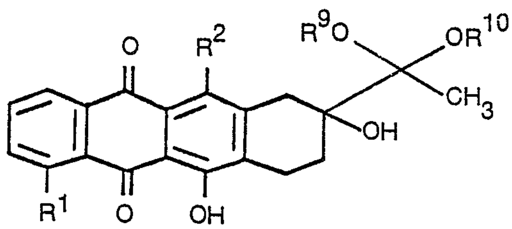

(II)

wherein $R^9$ and $R^{10}$ are each a lower alkyl group or, when taken together, represent an ethylene group and $R^1$ and $R^2$ are each as defined above with a halogenating agent to give the 7—X compound of the formula:

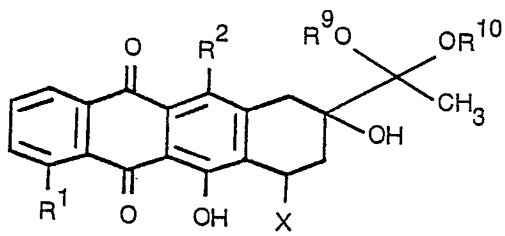

(III)

wherein X is a halogen atom and $R^1$, $R^2$, $R^9$ and $R^{10}$ are each as defined above and reacting the latter with an amine of the formula:

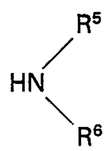

(C)

wherein $R^5$ and $R^6$ are each as defined above to give the 7-amino compound of the formula:

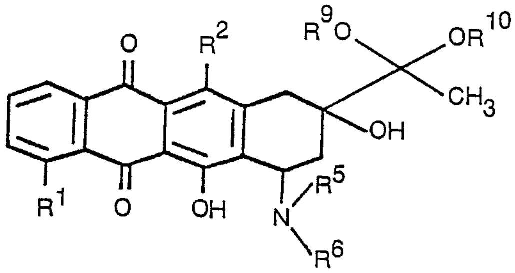

(Ia)

wherein $R^1$, $R^2$, $R^5$, $R^6$, $R^9$ and $R^{10}$ are each as defined above, optionally followed by elimination of the acetal group at the 13-position to give the 7-amino compound of the formula:

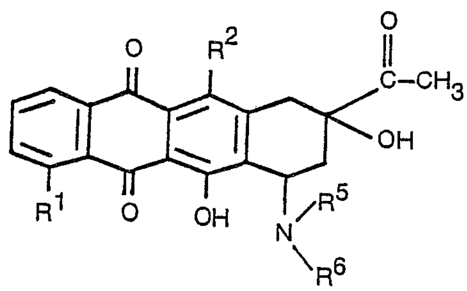

(Ib)

wherein $R^1$, $R^2$, $R^5$ and $R^6$ are each as defined above.

2. A process for preparing a compound of the formula:

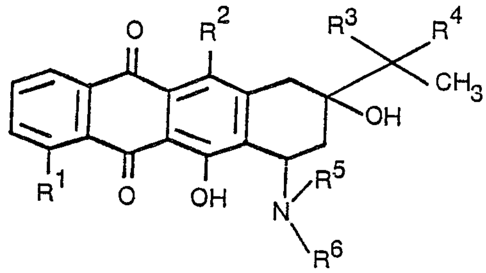

(I)

wherein $R^1$, $R^2$, $R^3$, $R^4$, $R^5$ and $R^6$ are each as defined in Claim 1 or its acid additional salt, which process comprises reacting a compound of the formula:

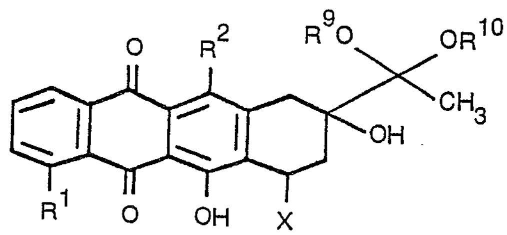

(III)

wherein X is a halogen atom, $R^9$ and $R^{10}$ are each a lower alkyl group or, when taken together, represent an ethylene group and $R^1$ and $R^2$ are each as defined above with an amine of the formula:

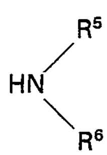

(C)

wherein $R^5$ and $R^6$ are each as defined above to give the 7-amino compound of the formula:

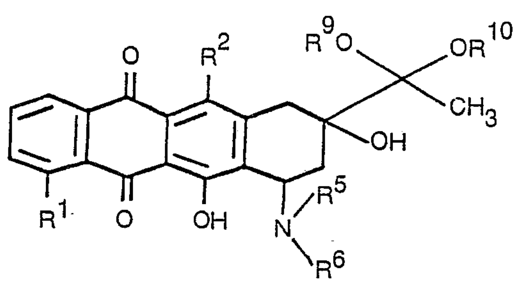

(Ia)

wherein $R^1$, $R^2$, $R^5$, $R^6$, $R^9$ and $R^{10}$ are each as defined above, optionally followed by elimination of the acetal group at the 13-position to give the 7-amino compound of the formula:

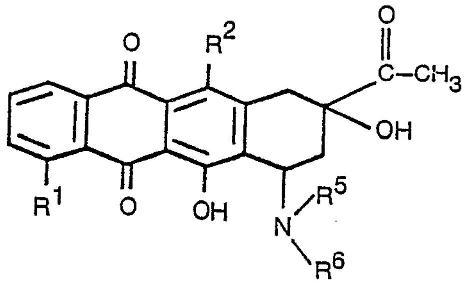

(Ib)

wherein $R^1$, $R^2$, $R^5$ and $R^6$ are each as defined above.

3. A process as claimed in Claim 1 or Claim 2 wherein $R^3$ and $R^4$ are each lower alkoxy or, when taken together, represent an ethylenedioxy group.

4. A process as claimed in Claim 1 or Claim 2 wherein $R^3$ and $R^4$ are taken together to represent an oxo group.

5. A process as claimed in Claim 1 or Claim 2 wherein $R^3$ and $R^4$ are taken together to represent an oxo group, $R^5$ is a hydrogen atom and $R^6$ is a group of the formula: —A—Q.

6. A process as claimed in Claim 1 or Claim 2 wherein $R^3$ and $R^4$ are taken together to represent an oxo group and $R^5$ is a hydrogen atom and $R^6$ is a group of the formula:

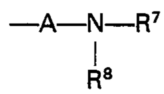

7. A process as claimed in Claim 1 or Claim 2 wherein $R^7$ and $R^8$ are each a hydrogen atom, a lower alkyl group, a benzyl group, a hydroxy(lower)alkyl group or an amino(lower)alkyl group.

8. A process according to Claim 5, wherein $R^7$ and $R^8$ are each a hydrogen atom or a lower alkyl group.

9. A compound as claimed in Claim 1 or Claim 2 wherein $R^1$ is a hydrogen atom and $R^2$ is a hydroxyl group.

10. A process as claimed in Claim 1 or Claim 2 wherein $R^1$ is a hydroxyl group and $R^2$ is a hydroxyl group.

**Patentansprüche für die Vertragsstaaten: BE CH DE FR GB IT LI NL SE**

1. Verbindung der Formel

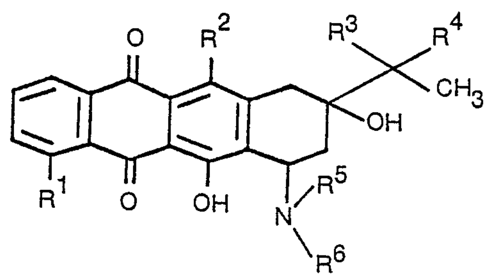

(I)

worin $R^1$ ein Wasserstoffatom, eine Hydroxylgruppe oder eine Niederigalkoxygruppe bedeutet, $R^2$ ein Wasserstoffatom oder eine Hydroxylgruppe bedeutet, $R^3$ und $R^4$ jeweils eine Niedrigalkoxygruppe oder gemeinsam eine Äthylendioxygruppe oder eine Oxogruppe bedeuten und $R^5$ und $R^6$ jeweils ein Wasserstoffatom, eine Niedrigalkylgruppe oder eine Gruppe der Formel —A—Q (worin A eine Alkylengruppe, die wahlweise zumindest ein Niedrigalkyl trägt, bedeutet und Q für ein Wasserstoffatom, eine Hydroxylgruppe, eine Niedrigalkoxygruppe oder eine Gruppe der Formel

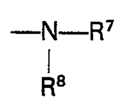

steht, worin $R^7$ und $R^8$ jeweils ein Wasserstoffatom, eine Niedrigalkylgruppe, eine Benzylgruppe, eine Hydroxy(niedrig)alkylgruppe oder eine Amino(niedrig)alkylgruppe bedeuten oder gemeinsam mit dem angrenzenden Stickstoffatom, an das sei gebunden sind, einen 5- bis 7-gliedrigen, Stickstoff enthaltenden Ring darstellen, welcher wahlweise ein zusätzliches Stickstoff- oder Sauerstoffatom im Ring aufweist und wahlweise irgendeinen Substituenten am Ring trägt) bedeuten, oder $R^5$ und $R^6$ gemeinsam mit dem angrenzenden Stickstoffatom, an welches sie gebunden sind, einen 5- bis 7-gliedrigen, Stickstoff enthaltenden Ring bedeuten, welcher wahlweise ein zusätzliches Stickstoff- oder Sauerstoffatom im Ring aufweist und wahlweise irgendeinen Substituenten am Ring trägt, oder deren Säureadditionssalz.

2. Verbindung nach Anspruch 1, worin $R^3$ und $R^4$ jeweils Niedrigalkoxy oder gemeinsam eine Äthylendioxygruppe bedeuten.

3. Verbindung nach Anspruch 1, worin $R^3$ und $R^4$ gemeinsam eine Oxogruppe bedeuten.

4. Verbindung nach Anspruch 1, worin $R^3$ und $R^4$ gemeinsam eine Oxogruppe bedeuten, $R^5$ ein Wasserstoffatom bedeutet und $R^6$ für eine Gruppe der Formel —A—Q steht.

5. Verbindung nach Anspruch 1, worin $R^3$ und $R^4$ gemeinsam eine Oxogruppe bedeuten und $R^5$ ein Wasserstoffatom bedeutet und $R^6$ für eine Gruppe der Formel

$$-A-\underset{\displaystyle R^8}{\underset{|}{N}}-R^7$$

steht.

6. Verbindung nach Anspruch 5, worin $R^7$ und $R^8$ jeweils ein Wasserstoffatom, eine Niedrigalkylgruppe, eine Benzylgruppe, eine Hydroxy(niedrig)alkylgruppe oder eine Amino(niedrig)alkylgruppe bedeuten.

7. Verbindung nach Anspruch 5, worin $R^7$ und $R^8$ jeweils ein Wasserstoffatom oder eine Niedrigalkylgruppe bedeuten.

8. Verbindung nach Anspruch 6, worin $R^1$ ein Wasserstoffatom bedeutet und $R^2$ für eine Hydroxylgruppe steht.

9. Verbindung nach Anspruch 6, worin $R^1$ eine Hydroxylgruppe bedeutet und $R^2$ eine Hydroxylgruppe bedeutet.

10. Verbindung nach irgendeinem der Ansprüche 1 bis 9, dadurch gekennzeichnet, daß die Konfigurationen an den Positionen 7 und 9 die S-Konfigurationen sind.

11. Verfahren zur Herstellung einer Verbindung der Formel

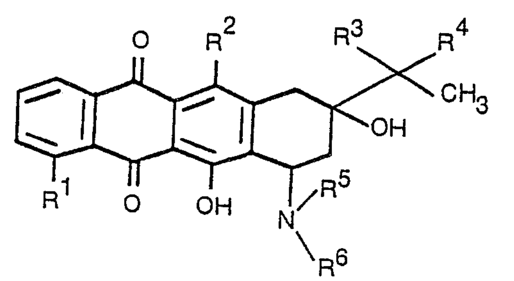

(I)

worin $R^1$, $R^2$, $R^3$, $R^4$, $R^5$ und $R^6$ der in Anspruch 1 angegebenen Definition entsprechen, oder ihres Säureadditionssalzes, welches Verfahren dadurch gekennzeichnet ist, daß eine Verbindung der Formel

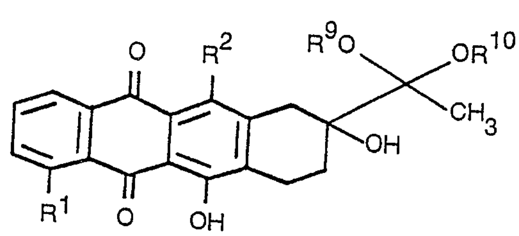

(II)

worin $R^9$ und $R^{10}$ jeweils eine Niedrigalkylgruppe oder gemeinsam eine Äthylengruppe bedeuten und $R^1$ und $R^2$ jeweils der voranstehenden Definition entsprechen, mit einem Halogenierungsmittel umgesetzt wird, um die 7-X-Verbindung der Formel

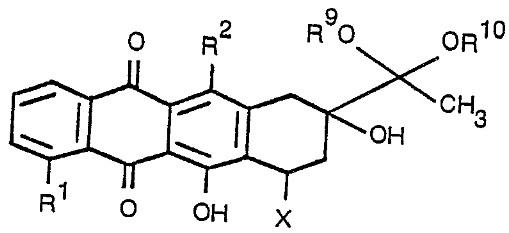

(III)

worin X ein Halogenatom bedeutet und $R^1$, $R^2$, $R^9$ und $R^{10}$ der voranstehenden Definition entsprechen, zu ergeben und letztere mit einem Amin der Formel

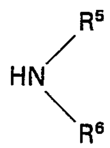

(C)

worin $R^5$ und $R^6$ der voranstehenden Definition entsprechen, umgesetzt wird, um die 7-Amino-Verbindung der Formel

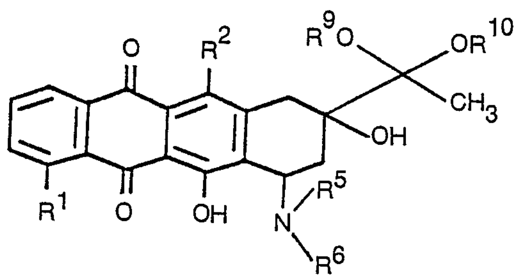

(Ia)

worin $R^1$, $R^2$, $R^5$, $R^6$, $R^9$ und $R^{10}$ jeweils obige Bedeutung haben, zu ergeben, wahlweise gefolgt von einer Eliminierung der Acetalgruppe an Position 13, was die 7-Amino-Verbindung der Formel

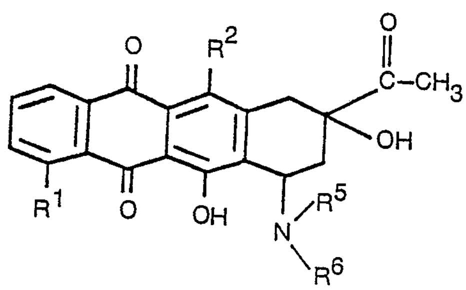

(Ib)

worin $R^1$, $R^2$, $R^5$ und $R^6$ jeweils die obige Bedeutung haben, ergibt.

12. Verfahren zur Herstellung einer Verbindung der Formel

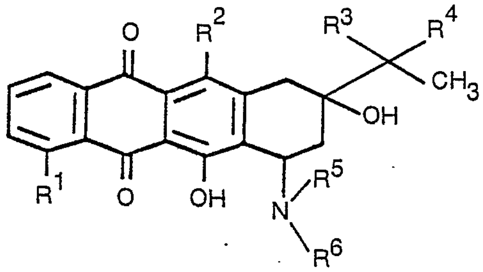

(I)

worin $R^1$, $R^2$, $R^3$, $R^4$, $R^5$ und $R^6$ der in Anspruch 1 angegebenen Definition entsprechen, oder ihres Säureadditionssalzes, welches Verfahren dadurch gekennzeichnet ist, daß eine Verbindung der Formel

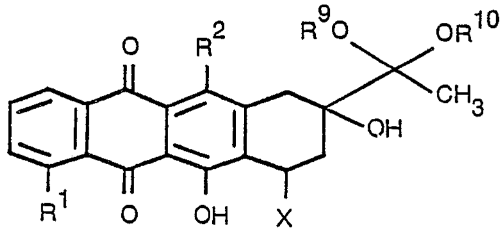

(III)

worin X ein Halogenatom bedeutet, $R^9$ und $R^{10}$ jeweils eine Niedrigalkylgruppe oder gemeinsam eine Äthylengruppe bedeuten und $R^1$ und $R^2$ die obige Bedeutung haben, mit einem Amin der Formel

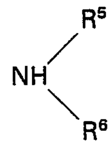

(C)

worin $R^5$ und $R^6$ die obige Bedeutung haben, umgesetzt wird, was die 7-Amino-Verbindung der Formel

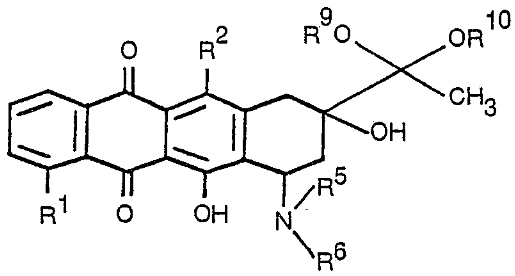

(Ia)

worin $R^1$, $R^2$, $R^5$, $R^6$, $R^9$ und $R^{10}$ jeweils der obigen Definition entsprechen, ergibt, gegebenenfalls gefolgt von einer Eliminierung der Acetalgruppe an Position 13, was die 7-Amino-Verbindung der Formel

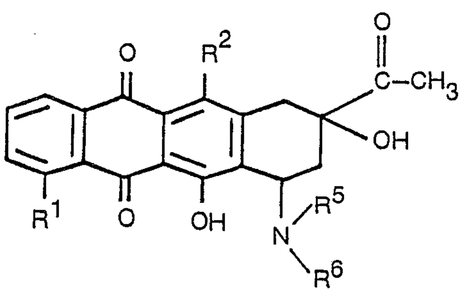

(Ib)

worin $R^1$, $R^2$, $R^5$ und $R^6$ jeweils der obigen Definition entsprechen, ergibt.

**Patentansprüche für den Vertragsstaat: AT**

1. Verfahren zur Herstellung einer Verbindung der Formel

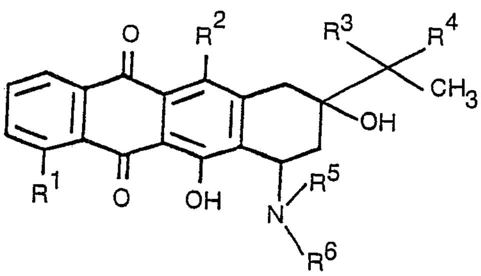

(I)

worin $R^1$ ein Wasserstoffatom, eine Hydroxylgruppe oder eine Niederigalkoxygruppe bedeutet, $R^2$ ein Wasserstoffatom oder eine Hydroxylgruppe bedeutet, $R^3$ und $R^4$ jeweils eine Niedrigalkoxygruppe oder gemeinsam eine Äthylendioxygruppe oder eine Oxogruppe bedeuten und $R^5$ und $R^6$ jeweils ein Wasserstoffatom, eine Niedrigalkylgruppe oder eine Gruppe der Formel —A—Q (worin A eine Alkylengruppe, die wahlweise zumindest ein Niedrigalkyl trägt, bedeutet und Q für ein Wasserstoffatom, eine Hydroxylgruppe, eine Niedrigalkoxygruppe oder eine Gruppe der Formel

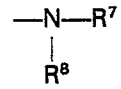

steht, worin $R^7$ und $R^8$ jeweils ein Wasserstoffatom, eine Niedrigalkylgruppe, eine Benzylgruppe, eine Hydroxyl(niedrig)alkylgruppe oder eine Amino(niedrig)alkylgruppe bedeuten oder gemeinsam mit dem angrenzenden Stickstoffatom, an das sie gebunden sind, einen 5- bis 7-gliedrigen, Stickstoff enthaltenden Ring darstellen, welcher wahlweise ein zusätzliches Stickstoff- oder Sauerstoffatom im Ring aufweist und wahlweise irgendeinen Substituenten am Ring trägt) bedeuten, oder $R^5$ und $R^6$ gemeinsam mit dem angrenzenden Stickstoffatom, an welches sie gebunden sind, einen 5- bis 7-gliedrigen, Stickstoff enthaltenden Ring bedeuten, welcher wahlweise ein zusätzliches Stickstoff- oder Sauerstoffatom im Ring aufweist und wahlweise irgendeinen Substituenten am Ring trägt, oder deren Säureadditionssalz, welches Verfahren dadurch gekennzeichnet ist, daß eine Verbindung der Formel

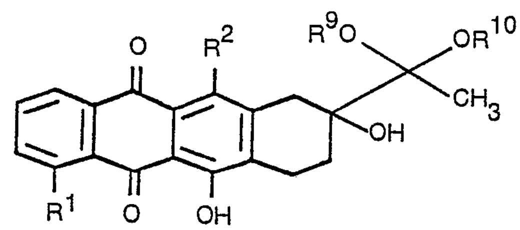

(II)

worin $R^9$ und $R^{10}$ jeweils eine Niedrigalkylgruppe oder gemeinsam eine Äthylengruppe bedeuten und $R^1$ und $R^2$ jeweils der voranstehenden Definition entsprechen, mit einem Halogenierungsmittel umgesetzt wird, um die 7-X-Verbindung der Formel

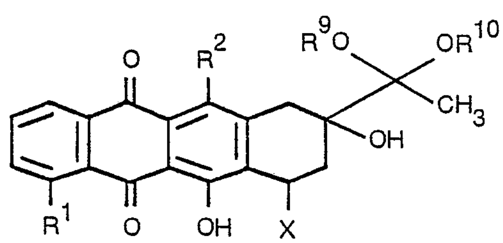

(III)

worin X ein Halogenatom bedeutet und $R^1$, $R^2$, $R^9$ und $R^{10}$ jeweils der voranstehenden Definition entsprechen, zu ergeben und letztere mit einem Amin der Formel

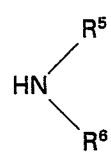

(C)

worin $R^5$ und $R^6$ jeweils der voranstehenden Definition entsprechen, umgesetzt wird, um die 7-Amino-Verbindung der Formel

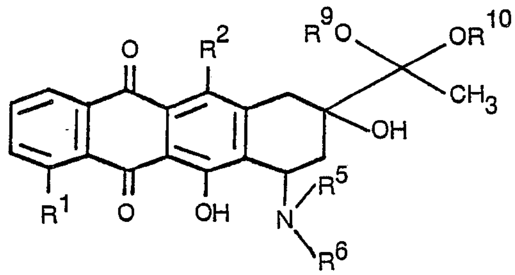

(Ia)

worin $R^1$, $R^2$, $R^5$, $R^6$, $R^9$ und $R^{10}$ jeweils der voranstehenden Definition entsprechen, zu ergeben, wahlweise gefolgt von einer Eliminierung der Acetalgruppe an Position 13, was die 7-Amino-Verbindung der Formel

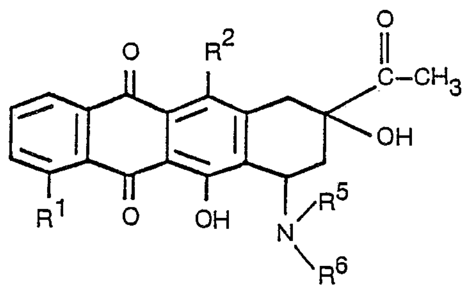

(Ib)

worin $R^1$, $R^2$, $R^5$ und $R^6$ jeweils der voranstehenden Definition entsprechen, ergibt.

2. Verfahren zur Herstellung einer Verbindung der Formel

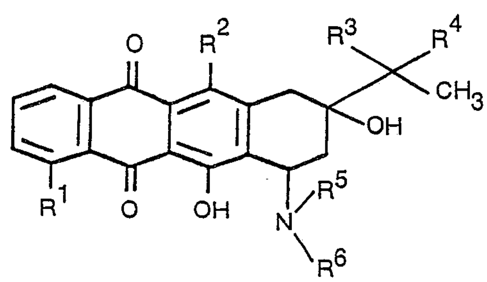

(I)

worin $R^1$, $R^2$, $R^3$, $R^4$, $R^5$ und $R^6$ jeweils der in Anspruch 1 angegebenen Definition entsprechen, oder ihres Säureadditionssalzes, welches Verfahren dadurch gekennzeichnet ist, daß eine Verbindung der Formel

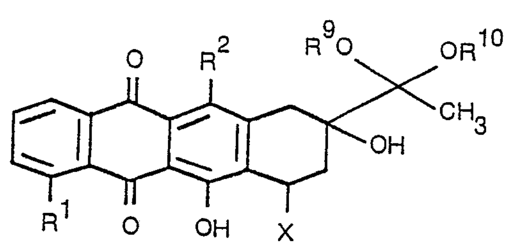

(III)

worin X ein Halogenatom bedeutet, $R^9$ und $R^{10}$ jeweils eine Niedrigalkylgruppe oder gemeinsam eine Äthylengruppe bedeuten und $R^1$ und $R^2$ jeweils der voranstehenden Definition entsprechen, mit einem Amin der Formel

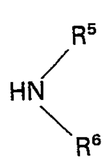

(C)

worin $R^5$ und $R^6$ jeweils der voranstehenden Definition entsprechen, umgesetzt wird, was die 7-Amino-Verbindung der Formel

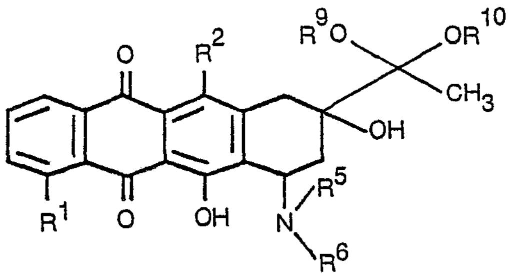

(Ia)

worin $R^1$, $R^2$, $R^5$, $R^6$, $R^9$ und $R^{10}$ jeweils der obigen Definition entsprechen, ergibt, gegebenenfalls gefolgt von einer Eliminierung der Acetalgruppe an Position 13, was die 7-Amino-Verbindung der Formel

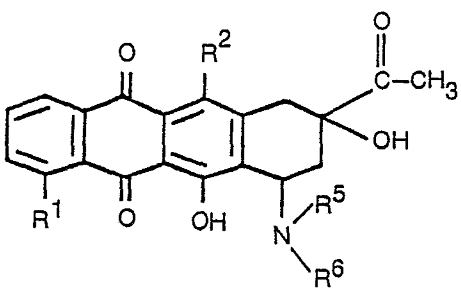

(Ib)

worin $R^1$, $R^2$, $R^5$ und $R^6$ jeweils der obigen Definition entsprechen, ergibt.

3. Verfahren nach Anspruch 1 oder Anspruch 2, dadurch gekennzeichnet, daß $R^3$ und $R^4$ jeweils Niedrigalkoxy bedeuten oder gemeinsam eine Äthylendioxygruppe darstellen.

4. Verfahren nach Anspruch 1 oder Anspruch 2, dadurch gekennzeichnet, daß $R^3$ und $R^4$ gemeinsam eine Oxogruppe bedeuten.

5. Verfahren nach Anspruch 1 oder Anspruch 2, dadurch gekennzeichnet, daß $R^3$ und $R^4$ gemeinsam eine Oxogruppe bedeuten, $R^5$ ein Wasserstoffatom bedeutet und $R^6$ eine Gruppe der Formel —A—Q darstellt.

6. Verfahren nach Anspruch 1 oder Anspruch 2, dadurch gekennzeichnet, daß $R^3$ und $R^4$ gemeinsam eine Oxogruppe darstellen, $R^5$ ein Wasserstoffatom bedeutet und $R^6$ eine Gruppe der Formel

$$—A—\underset{\displaystyle R^8}{\underset{|}{N}}—R^7$$

bedeutet.

7. Verfahren nach Anspruch 1 oder Anspruch 2, dadurch gekennzeichnet, daß $R^7$ und $R^8$ jeweils ein Wasserstoffatom, eine Niedrigalkylgruppe, eine Benzylgruppe, eine Hydroxy(niedrig)alkylgruppe oder eine Amino(niedrig)alkylgruppe bedeuten.

8. Verfahren nach Anspruch 5, dadurch gekennzeichnet, daß $R^7$ und $R^8$ jeweils ein Wasserstoffatom oder eine Niedrigalkylgruppe bedeuten.

9. Verfahren nach Anspruch 1 oder Anspruch 2, dadurch gekennzeichnet, daß $R^1$ ein Wasserstoffatom bedeutet und $R^2$ für eine Hydroxylgruppe steht.

10. Verfahren nach Anspruch 1 oder Anspruch 2, dadurch gekennzeichnet, daß $R^1$ eine Hydroxylgruppe bedeutet und $R^2$ eine Hydroxylgruppe bedeutet.

**Revendications pour les Etats contractants: BE CH DE FR GB IT LI NL SE**

1. Un composé de formule:

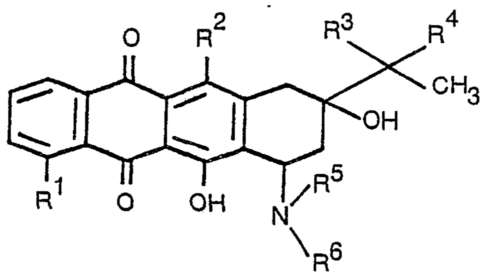

(I)

dans laquelle $R^1$ représente un atome d'hydrogène, un groupe hydroxyle ou un groupe alcoxy inférieur, $R^2$ représente un atome d'hydrogène ou un groupe hydroxyle, $R^3$ et $R^4$ représentent chacun un groupe alcoxy inférieur ou, pris ensemble, représentent un groupe éthylènedioxy ou un groupe oxo et $R^5$ et $R^6$ représentent chacun un atome d'hydrogène, un groupe alcoyle inférieur ou un groupe de formule: —A—Q (dans laquelle A représente un groupe alcoylène qui porte éventuellement au moins un groupe alcoyle inférieur et Q représente un atome d'hydrogène, un groupe hydroxyle, un groupe alcoxy inférieur ou un groupe de formule:

$$-\underset{\displaystyle R^8}{\underset{|}{N}}-R^7$$

dans laquelle $R^7$ et $R^8$ représentent chacun un atome d'hydrogène, un groupe alcoyle inférieur, un groupe benzyle, un groupe hydroxyalcoyle inférieur ou un groupe aminoalcoyle inférieur ou, pris ensemble avec l'atome d'hydrogène adjacent auquel ils sont attachés, ils représentent un cycle azoté comportant de 5 à 7 chaînons, ledit cycle pouvant comprendre un atome d'azote ou d'oxygène supplémentaire et pouvant porter un substituant quelconque) ou $R^5$ et $R^6$ pris ensemble avec l'atome d'azote adjacent auquel ils sont attachés représentent un cycle azoté comportant de 5 à 7 chaînons, ledit cycle pouvant comprendre l'atome d'azote adjacent ou d'oxygène supplémentaire et pouvant porter un substituant quelconque, ou un sel d'addition d'acide de ce composé.

2. Un composé selon la revendication 1, dans lequel $R^3$ et $R^4$ représentent un groupe alcoxy inférieur ou, pris ensemble, représentent un groupe éthylènedioxy.

3. Un composé selon la revendication 1, dans lequel $R^3$ et $R^4$, pris ensemble, représentent un groupe oxo.

4. Un composé selon la revendication 1, dans lequel $R^3$ et $R^4$, pris ensemble, représentent un groupe oxo, $R^5$ représentent un atome d'hydrogène et $R^6$ représente un groupe de formule: —A—Q.

5. Un composé selon la revendication 1, dans lequel $R^3$ et $R^4$, pris ensemble, représentent un groupe oxo et $R^5$ représente un atome d'hydrogène et $R^6$ un groupe de formule:

$$-A-\underset{\displaystyle R^8}{\underset{|}{N}}-R^7$$

6. Un composé selon la revendication 5, dans lequel $R^7$ et $R^8$ représentent chacun un atome d'hydrogène, un groupe alcoyle inférieur, un groupe benzyle, un groupe hydroxyalcoyle inférieur ou un groupe aminoalcoyle inférieur.

7. Un composé selon la revendication 5, dans lequel $R^7$ et $R^8$ représentent chacun un atome d'hydrogène ou un groupe alcoyle inférieur.

8. Un composé selon la revendication 6, dans lequel $R^1$ représente un atome d'hydrogène et $R^2$ représente un groupe hydroxyle.

9. Un composé selon la revendication 6, dans lequel $R^1$ représente un groupe hydroxyle et $R^2$ représente un groupe hydroxyle.

10. Un composé selon l'une quelconque des revendications 1 à 9, dans lequel la configuration aux positions 7 et 9 est la configuration S.

11. Un procédé de préparation d'un composé de formule:

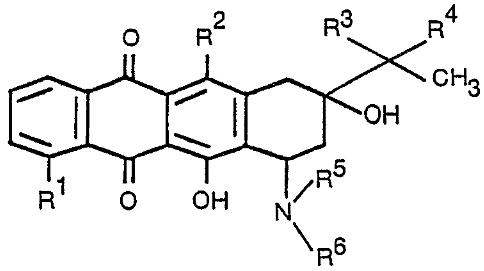

(I)

dans laquelle $R^1$, $R^2$, $R^3$, $R^4$, $R^5$ et $R^6$ sont tels que définis à la revendication 1, ou d'un sel d'addition d'acide de ce composé, caractérisé en ce que l'on fait réagir un composé de formule:

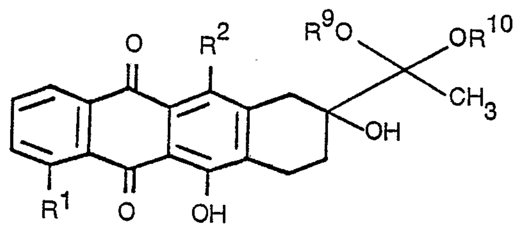

(II)

dans laquelle $R^9$ et $R^{10}$ représentent chacun un groupe alcoyle inférieur ou, pris ensemble, représentent un groupe éthylène et $R^1$ et $R^2$ sont chacun tels que définis ci-dessus, avec un agent d'halogénation, pour produire un composé 7-X de formule:

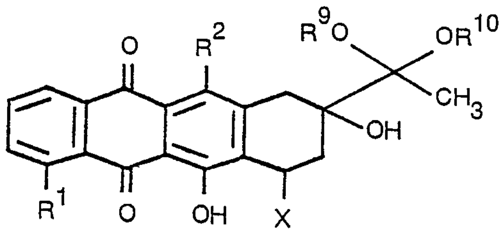

(III)

dans laquelle X représente un atome d'halogène et $R^1$, $R^2$, $R^9$ et $R^{10}$ sont chacun tels que définis ci-dessus, et on fait réagir ce dernier avec une amine de formule:

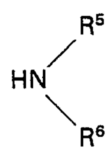

(C)

dans laquelle $R^5$ et $R^6$ sont chacun tels que définis ci-dessus, pour produire un composé 7-amino de formule:

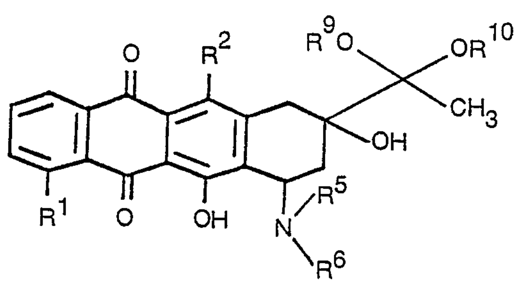

(Ia)

dans laquelle $R^1$, $R^2$, $R^5$, $R^6$, $R^9$ et $R^{10}$ sont chacun tels que définis ci-dessus, puis, si désiré, on élimine le groupe acétal à la position 13 pour produire un composé 7-amino de formule:

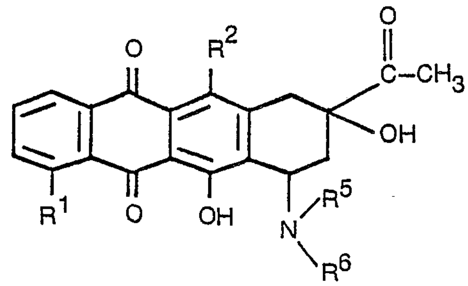

(Ib)

dans laquelle $R^1$, $R^2$, $R^5$ et $R^6$ sont chacun tels que définis ci-dessus.

12. Un procédé de préparation d'un composé de formule:

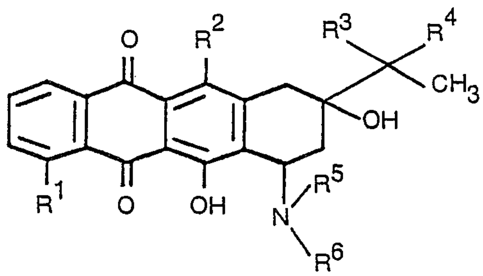

(I)

dans laquelle $R^1$, $R^2$, $R^3$, $R^4$, $R^5$ et $R^6$ sont tels que définis à la revendication 1, ou d'un sel d'addition d'acide de ce composé, caractérisé en ce que l'on fait réagir un composé de formule:

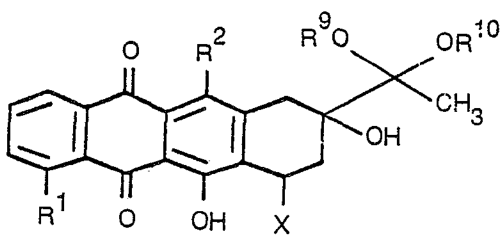

(III)

dans laquelle X représente un atome d'halogène, $R^9$ et $R^{10}$ représentent chacun un groupe alcoyle inférieur ou, pris ensemble, représentent un groupe éthylène et $R^1$ et $R^2$ sont chacun tels que définis ci-dessus, avec une amine de formule:

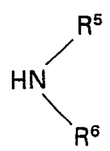

(C)

dans laquelle $R^5$ et $R^6$ sont chacun tels que définis ci-dessus, pour produire un composé 7-amino de formule:

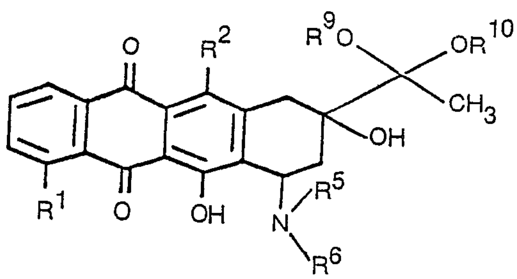

(Ia)

dans laquelle $R^1$, $R^2$, $R^5$, $R^6$, $R^9$ et $R^{10}$ sont chacun tels que définis ci-dessus, puis, si désiré, on élimine le groupe acétal à la position 13 pour produire un composé 7-amino de formule:

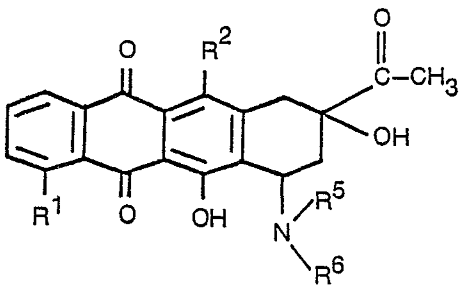
(Ib)

dans laquelle $R^1$, $R^2$, $R^5$ et $R^6$ sont chacun tels que définis ci-dessus.

**Revendications pour l'Etat contractant: AT**

1. Un procédé de préparation d'un composé de formule:

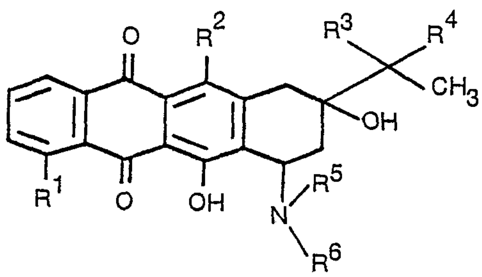
(I)

dans laquelle $R^1$ représente un atome d'hydrogène, un groupe hydroxyle ou un groupe alcoxy inférieur, $R^2$ représente un atome d'hydrogène ou un groupe hydroxyle, $R^3$ et $R^4$ représentent chacun un groupe alcoxy inférieur ou, pris ensemble, représentent un groupe éthylène dioxy ou un groupe oxo et $R^5$ et $R^6$ représentent chacun un atome d'hydrogène, un groupe alcoyle inférieur ou un groupe de formule: —A—Q (dans laquelle A représente un groupe alcoylène qui porte éventuellement au moins un groupe alcoyle inférieur et Q représente un atome d'hydrogène, un groupe hydroxyle, un groupe alcoxy inférieur ou un groupe de formule:

$$-N(R^8)-R^7$$

dans laquelle $R^7$ et $R^8$ représentent chacun un atome d'hydrogène, un groupe alcoyle inférieur, un groupe benzyle, un groupe hydroxyalcoyle inférieur ou un groupe aminoalcoyle inférieur ou, pris ensemble avec l'atome d'hydrogène adjacent auquel ils sont attachés, ils représentent un cycle azoté comportant de 5 à 7 chaînons, ledit cycle pouvant comprendre un atome d'azote ou d'oxygène supplémentaire et pouvant porter un substituant quelconque) ou $R^5$ et $R^6$ pris ensemble avec l'atome d'azote adjacent auquel ils sont attachés représentent un cycle azoté comportant de 5 à 7 chaînons, ledit cycle pouvant comprendre l'atome d'azote adjacent ou d'oxygène supplémentaire et pouvant porter un substituant quelconque, ou un sel d'addition d'acide de ce composé, ledit procédé étant caractérisé en ce que l'on fait réagir un composé de formule:

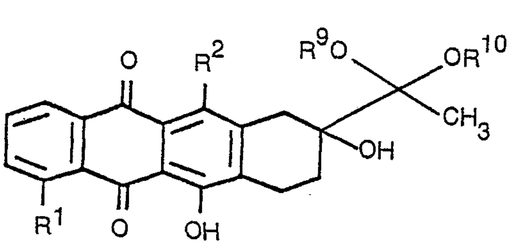
(II)

dans laquelle $R^9$ et $R^{10}$ représentent chacun un groupe alcoyle inférieur ou, pris ensemble, représentent un groupe éthylène et $R^1$ et $R^2$ sont chacun tels que définis ci-dessus, avec un agent d'halogénation, pour produire un composé 7-X de formule:

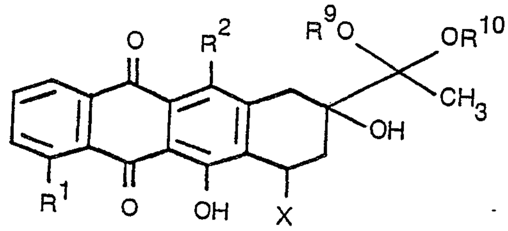

(III)

dans laquelle X représente un atome d'halogène et $R^1$, $R^2$, $R^9$ et $R^{10}$ sont chacun tels que définis ci-dessus, et on fait réagir ce dernier avec une amine de formule:

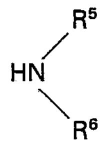

(C)

dans laquelle $R^5$ et $R^6$ sont chacun tels que définis ci-dessus, pour produire un composé 7-amino de formule:

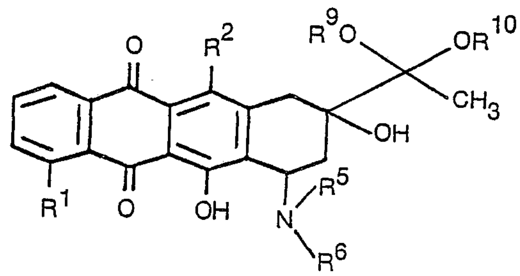

(Ia)

dans laquelle $R^1$, $R^2$, $R^5$, $R^6$, $R^9$ et $R^{10}$ sont chacun tels que définis ci-dessus, puis, si désiré, on élimine le groupe acétal à la position 13 pour produire un composé 7-amino de formule:

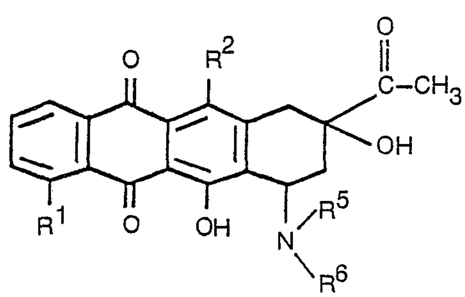

(Ib)

dans laquelle $R^1$, $R^2$, $R^5$ et $R^6$ sont chacun tels que définis ci-dessus.

2. Un procédé de préparation d'un composé de formule:

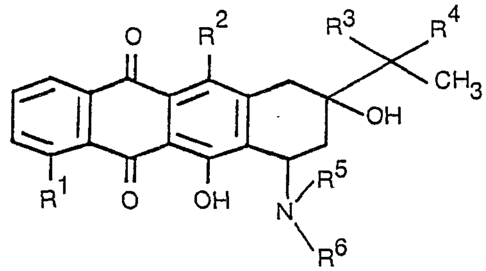

(I)

dans laquelle $R^1$, $R^2$, $R^3$, $R^4$, $R^5$ et $R^6$ sont chacun tels que définis à la revendication 1, ou d'un sel d'addition d'acide de ce composé, caractérisé en ce que l'on fait réagir un composé de formule:

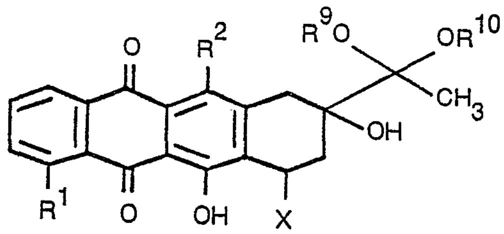

(III)

dans laquelle X représente un atome d'halogène, $R^9$ et $R^{10}$ représentent chacun un groupe alcoyle inférieur ou, pris ensemble, représentent un groupe éthylène et $R^1$ et $R^2$ sont chacun tels que définis ci-dessus, avec une amine de formule:

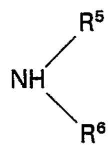

(C)

dans laquelle $R^5$ et $R^6$ sont chacun tels que définis ci-dessus, pour produire un composé 7-amino de formule:

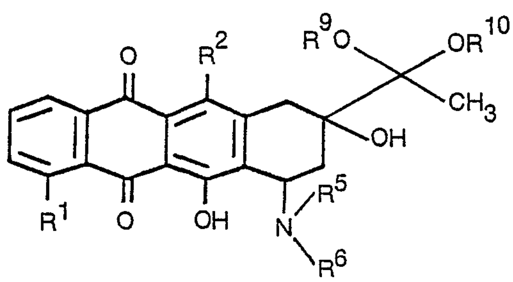

(Ia)

dans laquelle $R^1$, $R^2$, $R^5$, $R^6$, $R^9$ et $R^{10}$ sont chacun tels que définis ci-dessus, puis, si désiré, on élimine le groupe acétal à la position 13 pour produire un composé 7-amino de formule:

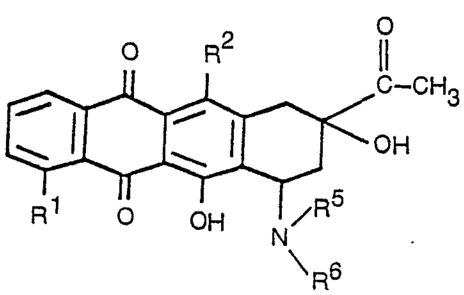

(Ib)

dans laquelle $R^1$, $R^2$, $R^5$ et $R^6$ sont chacun tels que définis ci-dessus.

3. Un procédé selon la revendication 1 ou la revendication 2, dans lequel $R^3$ et $R^4$ représentent chacun un groupe alcoxy inférieur ou, pris ensemble, représentent un groupe éthylènedioxy.

4. Un procédé selon la revendication 1 ou la revendication 2, dans lequel $R^3$ et $R^4$ pris ensemble, représentent un groupe oxo.

5. Un procédé selon la revendication 1 ou la revendication 2, dans lequel $R^3$ et $R^4$ pris ensemble, représentent un groupe oxo, $R^5$ représentent un atome d'hydrogène et $R^6$ représente un groupe de formule —A—Q.

6. Un procédé selon la revendication 1 ou la revendication 2, dans lequel $R^3$ et $R^4$, pris ensemble, représent un groupe oxo, $R^5$ représente un atome d'hydrogène et $R^6$ représente un groupe de formule

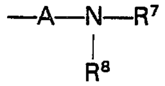

7. Un procédé selon la revendication 1 ou la revendication 2, dans lequel $R^7$ et $R^8$ représentent chacun un atome d'hydrogène, un groupe alcoyle inférieur, un groupe benzyle, un groupe hydroxy alcoyle inférieur ou un groupe amino alcoyle inférieur.

8. Le composé selon la revendication 5, dans lequel $R^7$ et $R^8$ représentent chacun un atome d'hydrogène ou un groupe alcoyle inférieur.

9. Un procédé selon la revendication 1 ou la revendication 2, dans lequel $R^1$ représente un atome d'hydrogène et $R^2$ représente un groupe hydroxyle.

10. Un procédé selon la revendication 1 ou la revendication 2, dans lequel $R^1$ représente un groupe hydroxyle et $R^2$ représente un groupe hydroxyle.